# EUROPEAN PATENT SPECIFICATION

(11) **EP 0 733 206 B1**
(45) Date of publication and mention of the grant of the patent: **22.04.1998**
(21) Application number: 95903781.3
(22) Date of filing: 30.11.1994
(51) Int. Cl.: G01N 33/533, C07K 14/00, C12Q 1/68, C07H 21/00

(54) **LABELLING OF NUCLEIC ACID ANALOGUE-PEPTIDE CHIMERAE**
MARKIEREN VON NUKLEINSÄUREANALOGEN PEPTID-CHIMÄREN
MARQUAGE DE CHIMERES ANALOGUE/PEPTIDE D'ACIDE NUCLEIQUE

(30) Priority: 06.12.1993 GB 9324956
(43) Date of publication of application: 25.09.1996
(73) Proprietor: PNA Diagnostics A/S, 2100 Copenhagen O (DK); Nielsen, Peter Eigil, Prof. Dr., 2980 Kokkedal (DK)
(72) Inventor: ORUM, Henrik, 3500 Vaerlose (DK); NIELSEN, Peter Eigil, Prof. Dr., 2980 Kokkedal (DK); STANLEY, Christopher John, Huntingdon, Cambridgeshire PE17 4JB (GB)
(74) Representative: Knauer, Martin, Dr.
(86) International application number: EP9403972
(87) International publication number: WO9516202

(56) References cited:
- WO-A-92/20793
- US-A- 4 923 802

## Description

The present invention relates to the production of labelled nucleic acid analogues and their use in analytical procedures.

Nucleic acid analogues having important new utilities in assay procedures and in the field of diagnostics have been described in WO 92/20703. These nucleic acid analogues had a number of new properties making them of special importance in the field of diagnostics as well as in the field of antisense therapeutics.

They typically feature a polyamide backbone bearing a sequence of ligands which are nucleic acid bases. The analogues described there have the property of hybridising with great specificity and stability to natural nucleic acids of complementary sequence.

In order to aid the detection and the manipulation of such nucleic acid analogues in diagnostic or other assay procedures and the like operations, it is desirable to provide the nucleic acid analogues with detectable labels.

In US-A-4,923,802, there is described an assay for protein kinase C by employing radiolabelled ATP and a highly specific peptide substrate composed in basic form of a serine or threonine amino acid residue flanked by groups of basic amino acids composed entirely of arginine, lysine or histidine or any combination of these amino acid residues. During the assay the phosphate group is transferred from ATP by the protein kinase C to the peptide. The presence of the label on the peptide is used as a measure for determining protein kinase C enzyme activity.

It has been proposed to radio-label such nucleic acid analogues. Other labelling techniques have been proposed also. We have now developed certain chimeric structures in which a nucleic acid analogue as previously described is linked to a peptide motif (i.e. a series of peptide bonded amino acids) selected such that the chimera undergoes a convenient labelling reaction.

Accordingly, the present invention provides in a first aspect, a method for labelling a nucleic acid analogue comprising providing a nucleic acid analogue with a peptide motif capable of functioning as a substrate for an enzyme in a labelling reaction and carrying out a said labelling reaction comprising reacting the peptide motif of the nucleic acid analogue under the influence of an enzyme with a source of said label. Preferably, said label is a radio-label, preferably radio-labelled ATP.

The radio-active atom in the radio-label is preferably ³²P at the γ position.

Alternatively, however the label may be any other detectable moiety which is attachable to a peptide in an enzyme mediated reaction, e.g. a biotin label.

The enzyme is preferably a protein kinase.

The peptide motif is preferably the kemptide motif, i.e. H-leu-Arg-Arg-Ala-Ser-Leu-Gly-. It is known that this motif when present in a protein or peptide will undergo a labelling reaction in which it acts as a substrate for the action of protein kinase A and is phosphorylated at the serine residue. Other phosphorylatable motifs may also be used. These include abbreviated kemptide motifs, e.g. the first five amino acid residues of the kemptide motif H-Arg-Ala-Ser-Leu-Gly-.

Preferably therefore, the labelling reaction is a phosphorylation at a serine residue of said peptide motif.

The nucleic acid analogue is preferably one comprising a polymeric strand which includes a sequence of ligands bound to a backbone made up of linked backbone moieties, which analogue is capable of hybridisation to a nucleic acid of complementary sequence, and further comprises said peptide motif.

Said nucleic acid analogue backbone is preferably a polyamide, polythioamide, polysulphinamide or polysulphonamide backbone.

Preferably, said linkded backbone moieties are peptide bonded amino acid moieties and preferably said peptide motif is present at the N-terminus or the C-terminus.

The nucleic acid analogue is preferably capable of hybridising to a nucleic acid of complementary sequence to form a hybrid which is more stable against denaturation by heat than a hybrid between the conventional deoxyribonucleotide corresponding in sequence to said analogue and said nucleic acid.

Preferably, said nucleic acid analogue is a peptide nucleic acid in which said backbone is a polyamide backbone, each said ligand being bonded directly or indirectly to an aza nitrogen atom in said backbone, and said ligand bearing nitrogen atoms mainly being separated from one another in said backbone by from 4 to 8 intervening atoms.

Preferably also, the nucleic acid analogue is capable of hybridising to a double stranded nucleic acid in which one strand has a sequence complementary to said analogue, in such a way as to displace the other strand from said one strand.

Preferably, the nucleic acid analogue has the general formula 1: 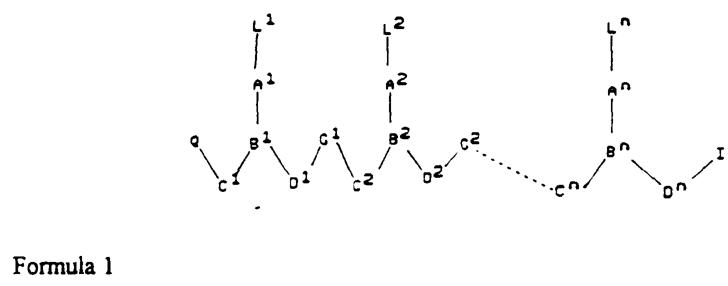 wherein:
n is at least 2,
each of L¹-Lⁿ is independently selected from the group consisting of hydrogen, hydroxy, (C₁-C₄)alkanoyl, naturally occurring nucleobases, non-naturally occurring nucleobases, aromatic moieties, DNA intercalators, nucleobase-binding groups, heterocyclic moieties, reporter ligands and said peptide motif, but normally at least one L will be a nucleobase binding group such as a naturally occurring nucleobase and preferably at least 90 % of the groups L will be such nucleobase binding groups;
each of C¹-Cⁿ is (CR⁶R⁷)y where R⁶ is hydrogen and R⁷ is selected from the group consisting of the side chains of naturally occurring alpha amino acids, or R⁶ and R⁷ are independently selected from the group consisting of hydrogen, (C₂-C₆)alkyl, aryl, aralkyl, heteroaryl, hydroxy, (C₁-C₆)alkoxy, (C₁-C₆)alkylthio, NR³R⁴ and SR⁵, where R³ and R⁴ are as defined below, and R⁵ is hydrogen, (C₁-C₆)alkyl or R⁶ and R⁷ taken together complete an alicyclic or heterocyclic system;
each of D¹-Dⁿ is (CR⁶R⁷)_{z} where R⁶ and R⁷ are as defined above;
each of y and z is zero or an integer from 1 to 10, the sum y + z being from 2 to 10 (preferably more than 2, and preferably such that each of x and y is 1 or 2);
each of G¹-Gⁿ⁻¹ is -NR³CO-, -NR³C^{S}-, -NR³SO- or -NR³SO₂-, in either orientation, where R³ is as defined below;
each of A¹-Aⁿ and B¹-Bⁿ are selected such that:
   (a) A is a group of formula (IIa), (IIb), (IIc) or (IId), and B is N or R³N⁺; or
   (b) A is a group of formula (IId) and B is CH; 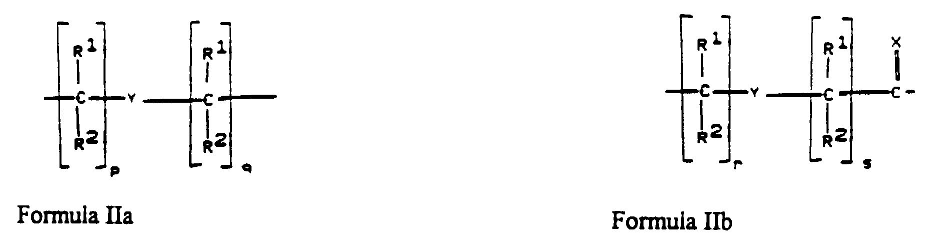 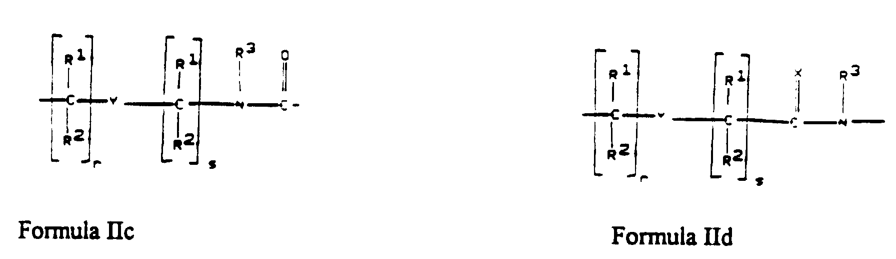
   wherein:
   X is O, S, Se, NR³, CH₂ or C(CH₃)₂;
   Y is a single bond, O, S or NR⁴;
each of p and q is zero or an integer from 1 to 5, the sum p + q being not more than 10;
each of r and s is zero or an integer from 1 to 5, the sum r + s being not more than 10;
each R¹ und R² is independently selected from the group consisting of hydrogen, (C₁-C₄)alkyl which may be hydroxy- or alkoxy- or alkylthio-substituted, hydroxy, alkoxy, alkylthio, amino and halogen; and
each R³ and R⁴ is independently selected from the group consisting of hydrogen, (C₁-C₄)alkyl, hydroxy- or alkoxy- or alkylthio-substituted (C₁-C₄)alkyl, hydroxy, alkoxy, alkylthio and amino;
Q is -CO₂H, -CONR'R", -SO₃H or -SO₂NR'R" or an activated derivative of -CO₂H or -SO₃H; and
I is -NR'R‴, where R' and R" are indenpendently selected from the group consisting of hydrogen, alkyl, amino protecting groups, reporter ligands, intercalators, chelators, peptides, proteins, carbohydrates, lipids, steroids, nucleosides, nucleotides, nucleotide diphosphates, nucleotide triphosphates, oligonucleotides, including both oligoribonucleotides and oligodeoxyribonucleotides, oligonucleosides and soluble and non-soluble polymers, and -R"' is an R" group or said peptide motif, at least one L group or -R‴ being said peptide motif.

More preferably, said nucleic acid analogue comprises a compound of the general formula m, IV or V: 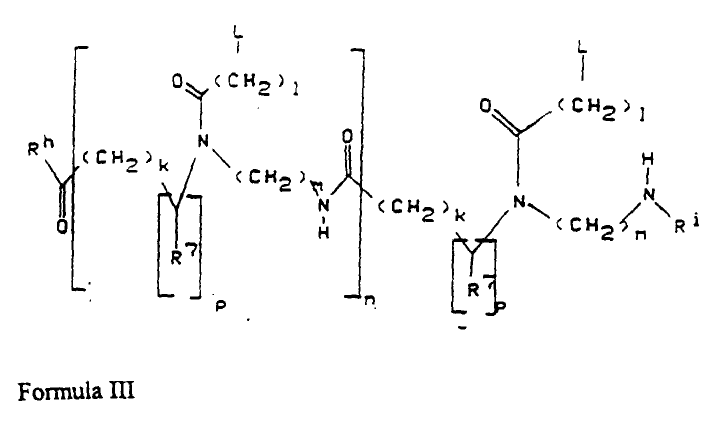 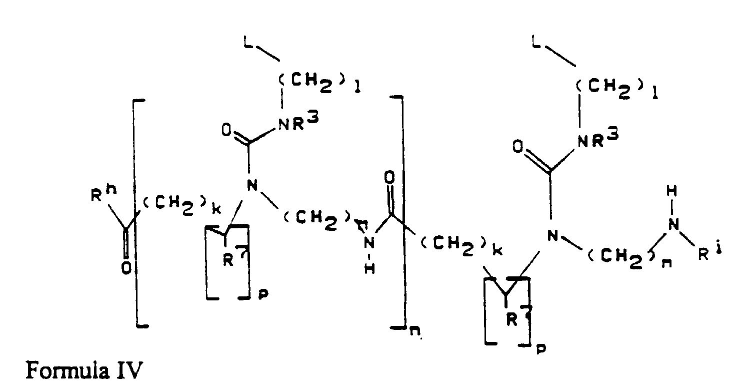 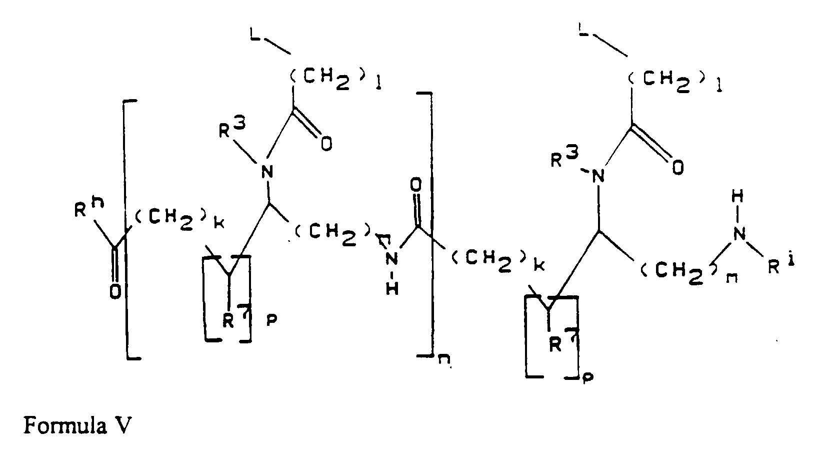 wherein:
each L is independently selected from the group consisting of hydrogen, phenyl, heterocyclic moieties, naturally occurring nucleobases, and non-naturally occurring nucleobases;
each R⁷ is independently selected from the group consisting of hydrogen and the side chains of naturally occurring alpha amino acids;
n is an integer greater than 1,
each k, l, and m is, independently, zero or an integer from 1 to 5;
each p is zero or 1;
R^{h} is OH, NH₂ or -NHLysNH₂; and
Rⁱ is said peptide motif.

Preferably the label is a ³²P label.

Preferably, the label is contained is a phosphate group attached to a serine residue which preferably forms part of a peptide motif.

The peptide motif preferably includes the kemptide motif.

In a third aspect, the invention provides a nucleic acid analogue comprising a polymeric strand which includes a sequence of ligands bound to a backbone made up of linked backbone moieties, which analogue is capable of hybridisation to a nucleic acid of complementary sequence, further comprising a peptide motif capable of acting as a substrate for an enzyme in a labelling reaction.

Preferably, said peptide motif is reactable with radio-labelled ATP to phosphorylate said peptide motif in the presence of a protein kinase and preferably therefore the peptide motif is the kemptide motif.

Preferably, the nucleic acid analogue is as described above in connection with the first aspect of the invention.

In a fourth aspect the invention provides an analytical method comprising hybridising a radio-labelled nucleic acid analogue produced by a method as described with reference to the first aspect of the invention or being in accordance with the second aspect of the invention, to a nucleic acid and detecting the presence of the hybrids so produced by the radio-label.

Preferably, the nucleic acid to be detected is bound to a support and is probed using said labelled nucleic acid analogue.

The invention will be further described and illustrated by the following description of preferred features of the invention and by the examples in which reference is made to the accompanying drawings in which:

Figure 1 is an autoradiograph produced in example 3 below.

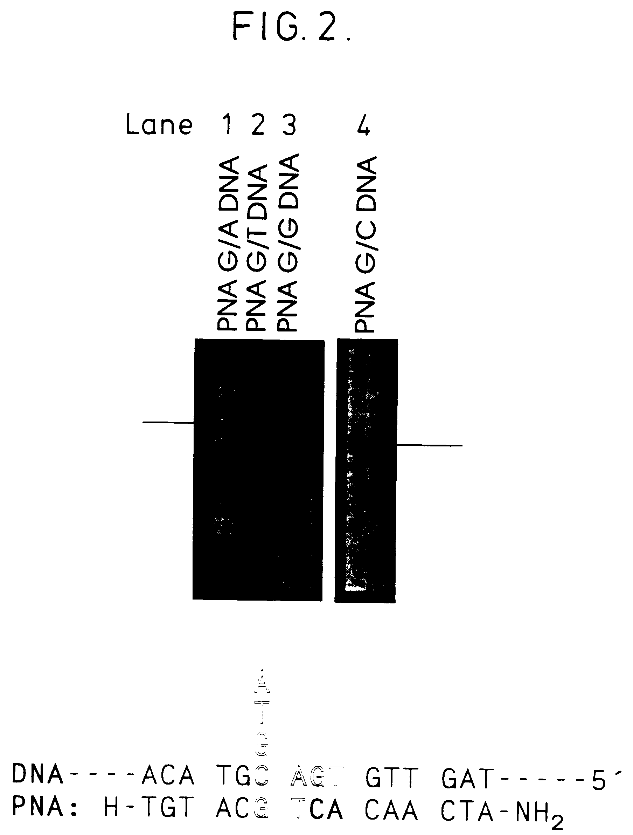

Figure 2 is an autoradiograph produced in example 4 below.

PNA's having peptide extensions of desired amino acid sequences can conveniently be produced by the Boc or Fmoc solid phase techniques well understood in the art once a starting PNA sequence has been built-up on a suitably solid support by using the Boc solid phase synthesis described in WO 92/20703. Alternatively, the peptide extension may be synthesised first before the PNA sequence is started.

The amino acid sequence of the peptide motif may be the kemptide motif for radio-labelling. For biotin labelling it may be a sequence as described in "Biotechnology", Vol 11, Oct. 1993, pp 1138-1142 by P.J. Schatz. Suitably the sequence is:

Leu-x-Leu-Zle-Phe-Glu-Ala-Gln-Lys-Zle-Glu-Trp-Arg which is biotinylated by the E. Coli biotin holoenzyme synthetase at the Lysine residue of biotin so supplied as biotinyl-5'-adenylate or biotin ad ATP.

The biotin can be used as a label detectable by avidin or streptavidin or may itself carry a radio-label such as ³H.

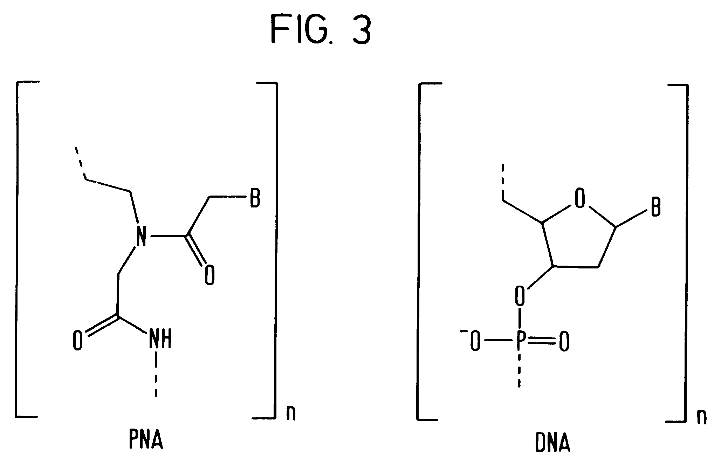

Figure 3 shows a structure of PNA (peptide nucleic acid) molecules compared to normal DNA.

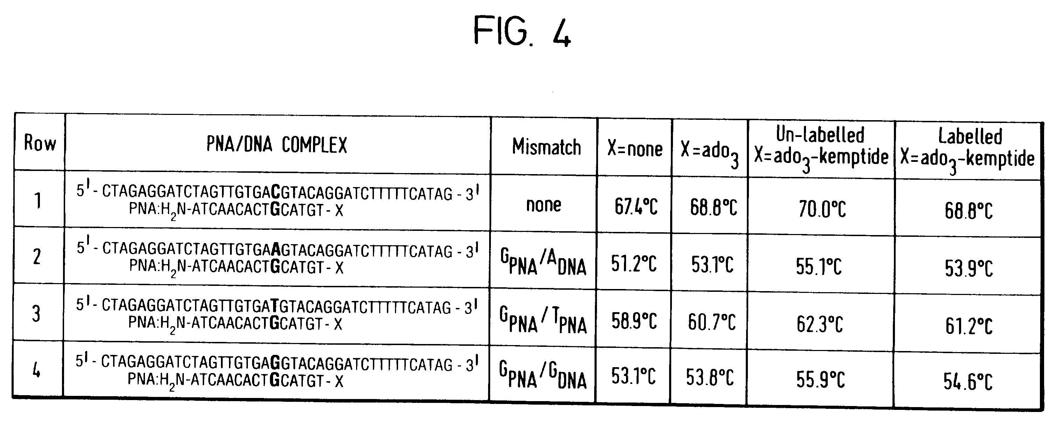

Figure 4 shows the sequence and nucleic acid hybridization characteristics of some of the PNA's used herein.

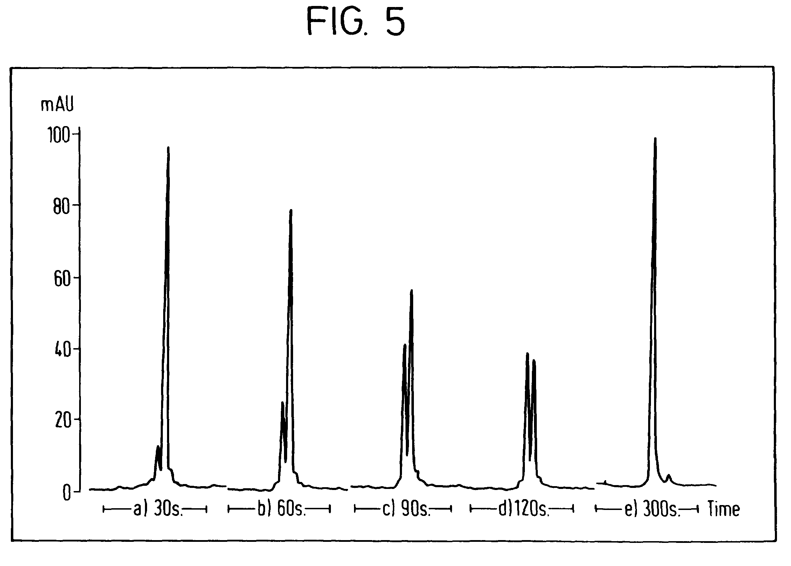

Figure 5 shows an HPLC analysis of a time study of the enzymatic labelling of the molecules according to the invention.

### Example 1

### Preparation of a PNA-kemptide chimera

The solid phase PNA synthesis described in WO 92/20703 was used to build up the sequence:

Boc-NH(CH2)5CONH-TG(Z)T.A(Z)C(Z)G(Z).TC(Z)A(Z).C(Z)A(Z)A(Z).C(Z)TA(Z)-
CONH-resin

The N terminal Boc group was removed by treatment with TFA and used as a starting point for a standard boc type solid phase peptide synthesis of the kemptide motif via the linker 6-amino-hexanoic acid to produce the chimera:

Boc-Leu-Arg(Tos)-Arg(Tos)-Ala-Ser-(Bzl)-Leu-Gly-NH(CH2)5-CONH-
TG(Z)T.A(Z)C(Z)G(Z).TC(Z)A(Z).C(Z)A(Z)   A(Z).C(Z)TA(Z)-CONH-resin

The protection groups were removed and the product cleaved from the resin by the Low-High TFMSA procedure. The raw product was purified by preparative HPLC (reversed phase C₁₈ eluting with a gradient of A:0. 1% TFA in water (MilliQ™) and B:0.1% TFA, 10% water, 90% acetonitrile). The purified chimeric PNA-kemptide was characterized by analytical HPLC and FAB-MS.

### Example 2

### The kemptide motif (Leu-Arg-Arg-Ala-Ser-Leu-Gly) functions as a substrate for protein kinase A covalently attached to a PNA

The PNA-kemptide chimera of the formula:
H-Leu-Arg-Arg-Ala-Ser-Leu-Gly-TGTACGTCACAACTA-NH₂ was labelled with ³²P in a reaction mixture containing:

| | |
|---|---|
| PNA-kemptide, 10 µM | 5 µl |
| 10 x Protein Kinase A buffer | 5 µl |
| γ ³²P ATP (>5000 Ci/mmol; 50 µCi/µl) | 10 µl |
| Protein Kinase A (Boehringer; 5 mU/µl) | 0.2 µl |
| H₂O | 30 µl |

The reaction was incubated for 30 minutes at 30°C and then for 10 minutes at 65°C before being centrifuged for 30 seconds at 15000 g. The supernatant was transferred to a new Eppendorff tube. Water was added to 1 ml and the labelled PNA-kemptide was separated from unincorporated γ ³²P ATP by anion exchange chromatography using a DEAE Sephadex™ A-50 anion exchange column.

The specific activity of the PNA-kemptide was estimated at 1 x 10⁸ cpm/µg PNA-kemptide.

### Example 3

### The hybridization properties of PNA are retained in a PNA-kemptide chimera

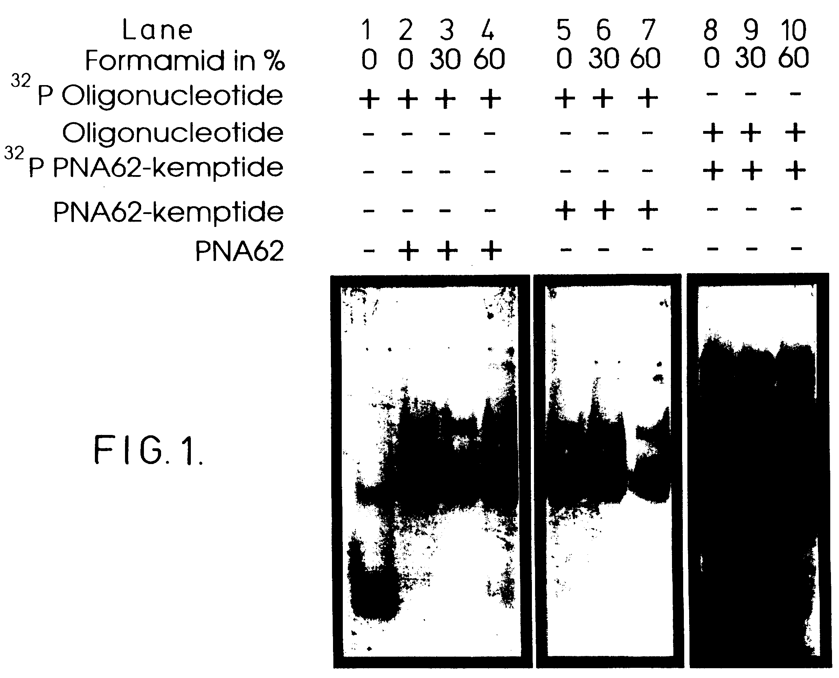

The abililty of unlabelled/³²P labelled PNA62-kemptide to hybridise to its complementary unlabelled/³²P labelled oligonucleotide in solution was analysed by gelshift in a 20% non-denaturing polyacrylamide gel. Hybridisation stringency was controlled by the addition of formamide, which suppresses the Tₘ of a PNA 62/DNA duplex at about ½°C/1% formamide. Hybridisations were carried out in a 20 µl reaction volume containing 10 mM Tris-Cl, pH 8.0, 1 mM EDTA, and PNA, DNA and formamide as indicated in the figure legend. Hybridisation mixtures were incubated for 15 min. at 37°C. At the end of the incubation period 4 µl loading buffer (50% glycerol, 5 x TBE buffer and 0.25% (w/v) bromphenolblue) was added to the reaction mix subsequent to which the samples were loaded on to a 20% non-denaturating polyacrylamide gel/1 x TBE and electrophoresed at 400 V for 1 hour. Finally the gel was subjected to autoradiography. Figure 1 shows the results of the hybridisation analyses. Lane 1: Labelled oligonucleotide alone (migration control). Lanes 2-4: Control PNA62 (PNA62 without the kemptide addition) incubated with labelled, complementary oligonculeotide in the presence of 0% (2), 30% (3) and 60% (4) formamide. Lanes 5-7: Unlabelled PNA62-kemptide incubated with labelled, complementary oligonucleotide in the presence of 0% (5), 30% (6) and 60% (7) formamide. Lanes 8-10: ³²P labelled PNA62-kemptide incubated with unlabelled, complementary oligonucleotide in the presence of 0% (5), 30% (6) and 60% (7) formamide. In conclusion these results show that:
1. The addition of the kemptide extension to PNA62 does not significantly alter its ability to hybridise with a complementary oligonucleotide.
2. Unlabelled and ³²P labelled PNA62-kemptide exhibits similar hybridisation properties.
3. A PNA carrying the kemptide motif is a substrate for phosphorylation by protein kinase A.

### Example 4

### ³²P labelled PNA62-kemptide can be used as a probe to detect complementary DNA fragments bound to a filter membrane

The labelled PNA62-kemptide was used as a probe to detect complementary sequences in a DNA fragment immobilised on a membrane (Southern hybridisation). DNA-framgents containing a sequence complementary to the PNA62-kemptide and DNA fragments containing a sequence with a single mismatch to the PNA62-kemptide were generated by PCR amplification of the appropriate plasmids. The DNA fragments were separated by gel-electrophoresis in a 1% TAE agarose gel and transferred to a Hybond™ N+ membrane by standard alkali blotting procedures. The filter was prehybridised at 50°C in a rotary oven in 5 ml hybridisation solution (10 mM Tris-HCl, pH 7.5, 1 mM EDTA) containing 4 ng unlabelled PNA T10-kemptide as a blocking agent for unspecific attachment of the probe to the filter. After 1 hour of prehybridisation, 5 µl of 32P labelled PNA62-kemptide was added to the prehybridisation solution and incubation was continued at 50°C for 16 hours. The filter was washed twice in 10 mM Tris-HCl pH 7½, 1 mM EDTA for 30 min. at 50°C, air dried, and exposed to autoradiography for 10 hours. As shown in figure 1, the PNA62-kemptide probe hybridises efficiently to the fully complementary DNA fragments on the filter (lane 4). Furthermore, it hybridises to some extent to the DNA fragment that carries the C to T point mutation (lane 2). This is expected because the PNA62 G/T DNA mismatched duplex in solution has a Tₘ of 61°C which is above the stringency level imposed on the hybridisation. In contrast to the G to T mutation, the C to G and C to A mutations have a major effect on the stability of the resulting PNA/DNA duplex in solution (PNA62 G/A DNA: Tm = 51°C and PNA62 G/G DNA: Tm = 53°C). Consistent with these Tₘ data, the PNA-kemptide probe does not hybridise to the corresponding DNA fragments on the filter (lane 1 and 3, respectively). In conclusion, the results shows that a labelled PNA-kemptide can be used as a probe in a filter hybridisation assay and that such probes are able to discriminate effectively between the fully complementary and single base mismatched target nucleic acids. Similar results were obtained using other membranes (Gene Screen™ + Immobilon S™) and other blocking reagents (1 % Casein and 1 %Triton™ X-100).

### Example 5

### Functional analysis of the peptide segment of the chimera

The chimera (20 pmol) and control DNA (Ado)₃-PNA) (20 pmol) were incubated separately in a reaction volume (50 µl) containg ³²P γ-ATP¹ (Amersham) (100 pmol > 5000 Ci/mmol), 50 mM MES (pH 6.9), 10 mM MgCl₂ 0.5 mM EDTA, 1 mM DTT, 1 mg/mL BSA and 5 mU PKA1 (Boehringer Mannheim). After 30 min at 30°C the PNAs were separated from unincorporated γ-ATP by ion exchange chromatography using diethylaminoethyl (DEAE) Sephadex A-50¹ (Sigma) and the purified PNAs were counted the control PNA. The sample containing the chimera had a specific activity of 2.4 x 10⁶ cpm/pmol PNA. When using ³²P γ-ATP at a specific acitivity of 5000 Ci/mmol the calculated maximum possible specific activity of the chimera would be 6 x 10⁶ dpm/pmol.

The enzymatic phosphorylation of the chimera was studied in a time course experiment. 1 mU PKA was used to phosphorylate 1 nmol chimera. Samples were taken at different time points during the reaction and analyzed on HPLC. Within 60 s ca. 20 % of the chimera had been phosphorylated (Fig. 5) and more than 50 % had been phosphorylated after 120 s. the reaction was completed within 300 s. The identity of the phosphorylated product was confirmed by mass spectroscopy (ESI; calculated/found: 5371.1/5370.9).

## Claims

1. A method for labelling a nucleic acid analogue comprising providing a nucleic acid analogue with a peptide motif capable of functioning as a substrate for an enzyme in a labelling reaction and carrying out a said labelling reaction comprising reacting the peptide motif of the nucleic acid analogue in a reaction mediated by said enzyme with a source of the label.

2. A method as claimed in claim 1, wherein said label is a radio-label.

3. A method as claimed in claim 1, wherein the source of said label is radio-labelled ATP.

4. A method as claimed in claim 3, wherein said enzyme is a protein kinase.

5. A method as claimed in any preceding claim, wherein the peptide motif is the kemptide motif.

6. A method as claimed in any one of claims 1 to 5, wherein the labelling reaction is phosphorylation at a serine residue of said peptide motif.

7. A method as claimed in any preceding claim, wherein the nucleic acid analogue comprises a polymeric strand which includes a sequence of ligands bound to a backbone made up of linked backbone moieties, which analogue is capable of hybridisation to a nucleic acid of complementary sequence, and further comprises said peptide motif.

8. A method as claimed in claim 7, wherein said nucleic acid analogue backbone is a polyamide, polythioamide, polysulphinamide or polysulphonamide backbone.

9. A method as claimed in claim 8, wherein said linked backbone moieties are peptide bonded amino acid moieties.

10. A method as claimed in claim 9, wherein said peptide motif is present at the N-terminus or is present at the C-terminus.

11. A method as claimed in any preceding claim, wherein the nucleic acid analogue is capable of hybridising to a nucleic acid of complementary sequence to form a hybrid which is more stable against denaturation by heat than a hybrid between the conventional deoxyribo-nucleotide corresponding in sequence to said analogue and said nucleic acid.

12. A method as claimed in any preceding claim, wherein said nucleic acid analogue is a peptide nucleic acid in which said backbone is a polyamide backbone, each said ligand being bonded directly or indirectly to an aza nitrogen atom in said backbone, and said ligand bearing nitrogen atoms mainly being separated from one another in said backbone by from 4 to 8 intervening atoms.

13. A method as claimed in any preceding claim, wherein the nucleic acid analogue is capable of hybridising to a double stranded nucleic acid in which one strand has a sequence complementary to said analogue, in such a way as to displace the other strand from said one strand.

14. A method as claimed in any preceding claim, wherein the nucleic acid analogue has the general formula 1: 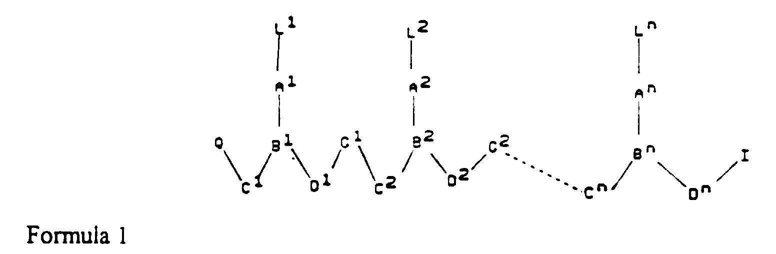 wherein:
n is at least 2,
each of L¹-Lⁿ is independently selected from the group consisting of hydrogen, hydroxy, (C₁-C₄)alkanoyl, naturally occurring nucleobases, non-naturally occurring nucleobases, aromatic moieties, DNA intercalators, nucleobase-binding groups, heterocyclic moieties, reporter ligands and said peptide motif;
each of C¹-Cⁿ is (CR⁶R⁷)_{y} where R⁶ is hydrogen and R⁷ is selected from the group consisting of the side chains of naturally occurring alpha amino acids, or R⁶ and R⁷ are independently selected from the group consisting of hydrogen, (C₂-C₆)alkyl, aryl, aralkyl, heteroaryl, hydroxy, (C₁-C₆)alkoxy, (C₁-C₆)alkylthio, NR³R⁴ and SR⁵, where R³ and R⁴ are as defined below, and R⁵ is hydrogen, (C₁-C₆)alkyl, hydroxy, alkoxy, or alkylthio-substituted (C₁to C₆)alkyl or R⁶ and R⁷ taken together complete an alicyclic or heterocyclic system;
each of D¹-Dⁿ is (CR⁶R⁷)_{z} where R⁶6 and R⁷ are as defined above;
each of y and z is zero or an integer from 1 to 10, the sum y + z being from 2 to 10;
each of G¹-Gⁿ is -NR³CO-, -NR³C^{S}-, -NR³SO- or -NR³ SO₂-, in either orientation, where R³ is as defined below;
each of A¹-Aⁿ and B¹-Bⁿ are selected such that:
(a) A is a group of formula (IIa), (IIb), IIc) or (IId), and B is N or R³N+; or
(b) A is a group of formula (IId) and B is CH; 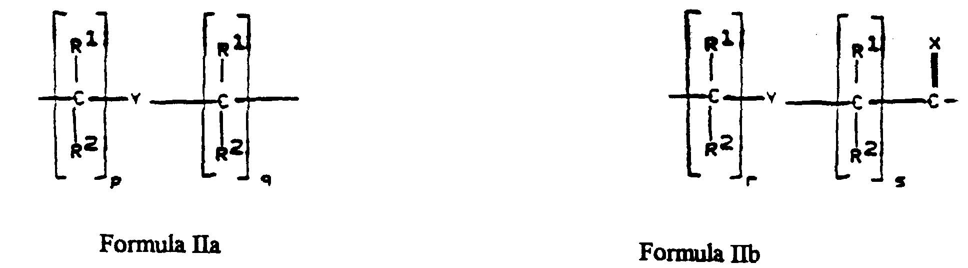 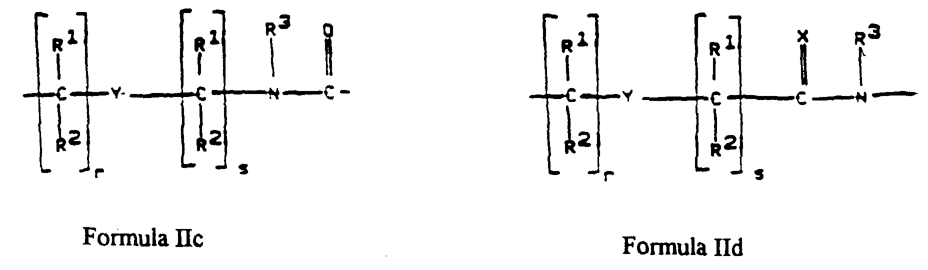
wherein:
X is O, S, Se, NR³, CH₂ or C(CH₃)₂;
Y is a single bond, O, S or NR⁴;
each of p an q is zero or an integer from 1 to 5, the sum p + q being not more than 10;
each of r and s is zero or an integer from 1 to 5, the sum r + s being not more than 10;
each of R¹ and R² is independently selected from the group consisting of hydrogen, (C₁-C₄)alkyl which may be hydroxy- or alkoxy- or alkylthio-substituted, hydroxy, alkoxy, alkylthio, amino and halogen; and
each R³ and R⁴ is independently selected from the group consisting of hydrogen, (C₁-C₄)alkyl, hydroxy- or alkoxy- or alkylthio-substituted (C₁-C₄)alkyl, hydroxy, alkoxy, alkylthio and amino;
Q is -CO₂H, -CONR'R", -SO₃H or -SO₂NR'R" or an activated derivative of -CO₂H or -SO₃H; and
I is -NR'R"', where R' and R" are independently selected from the group consisting of hydrogen, alkyl, amino protecting groups, reporter ligands, intercalators, chelators, peptides, proteins, carbohydrates, lipids, steroids, nucleosides, nucleotides, nucleotide diphosphates, nucleotide triphosphates, oligonucleotides, including both oligoribonucleotides and
oligodeoxyribonucleotides, oligonucleosides and soluble and non-soluble polymers, and -R"' is an R" group or said peptide motif, at least L or -R"' being said peptide motif.

15. A method as claimed in claim 14, wherein said nucleic acid analogue comprises a compound of the general formula III, IV or V: 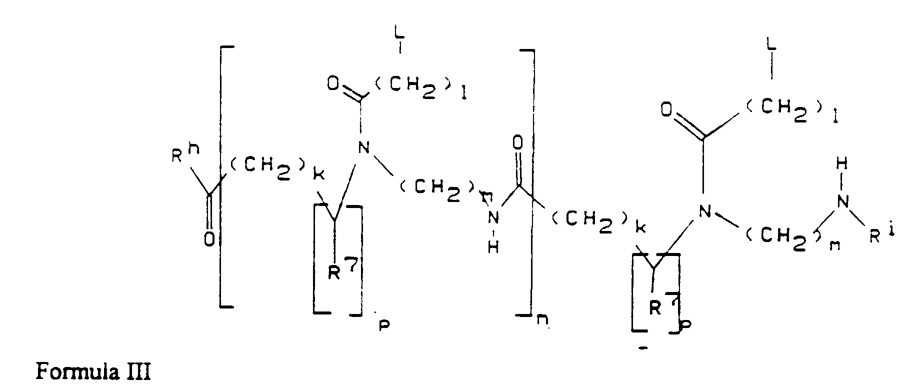 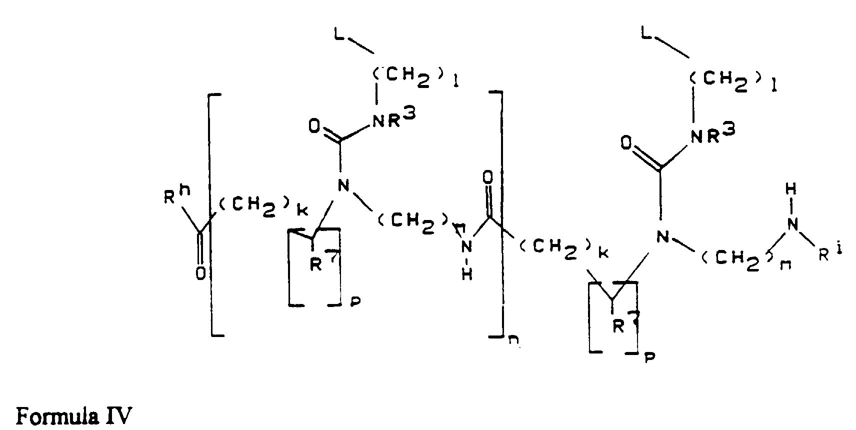 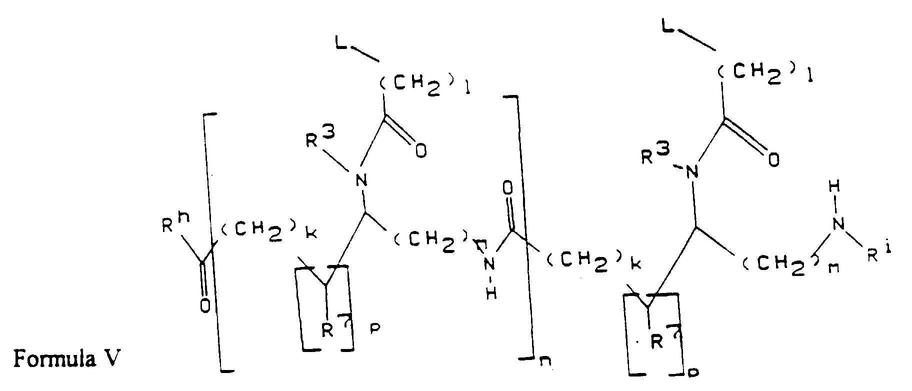 wherein:
each L is indenpendently selected from the group consisting of hydrogen, phenyl, heterocyclic moieties, naturally occurring nucleobases, and non-naturally occurring nucleobases;
each R⁷ is independently selected from the group consisting of hydrogen and the side chains of naturally occurring alpha amino acids;
n is an integer greater than 1,
each k, l, and m is, independently, zero or an integer from 1 to 5;
each p is zero or 1 ;
R^{h} is OH, NH₂ or -NHLysNH₂; and
R¹ is said peptide motif

16. A nucleic acid analogue comprising a polymeric strand which includes a sequence of ligands bound to a backbone made up of linked backbone moieties, which analogue is capable of hybridisation to a nucleic acid of complementary sequence, further comprising a peptide motif capable of acting as a substrate for an enzyme in a labelling reaction.

17. A nucleic acid analogue as claimed in claim 16, wherein said peptide motif is reactable with radio-labelled ATP to phosphorylate said peptide motif in the presence of a protein kinase.

18. A nucleic acid analogue as claimed in claim 17, wherein the peptide motif is the kemptide motif.

19. A nucleic acid analogue as claimed in any one of claims 16 to 18, wherein the backbone is a polyamide, polythioamide, polysulphinamide or polysulphonamide backbone.

20. A nucleic acid analogue as claimed in claim 19, wherein said linked backbone moieties are peptide bonded amino acid moieties.

21. A nucleic acid analogue as claimed in claim 19 or claim 20, wherein said peptide motif is present at the N-terminus or is present at the C-terminus.

22. A nucleic acid analogue as claimed in any one of claims 16 to 21, wherein the nucleic acid analogue is capable of hybridising to a nucleic acid of complementary sequence to form a hybrid which is more stable against denaturation by heat than a hybrid between the conventional deoxyribo-nucleotide corresponding in sequence to said analogue and said nucleic acid.

23. A nucleic acid analogue as claimed in any one of claims 16 to 22, wherein the nucleic acid analogue is a peptide nucleic acid in which said backbone is a polyamide backbone, each said ligand being bonded directly or indirectly to an aza nitrogen atom in said backbone, and said ligand bearing nitrogen atoms mainly being separated from one another in said backbone by from 4 to 8 intervening atoms.

24. A nucleic acid analogue as claimed in any one of claims 16 to 23, wherein the nucleic acid analogue is capable of hybridising to a double stranded nucleic acid in which one strand has a sequence complementary to said analogue, in such a way as to displace the other strand from said one strand.

25. A nucleic acid analogue as claimed in any one of claims 16 to 24, wherein the nucleic acid analogue has the general formula 1: 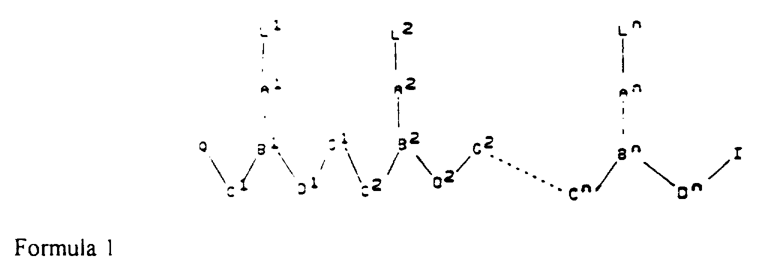 wherein
n is at least 2,
each of L¹-Lⁿ is independently selected from the group consisting of hydrogen, hydroxy, (C₁-C₄)alkanoyl, naturally occurring nucleobases, non-naturally occurring nucleobases, aromatic moieties, DNA intercalators, nucleobase-binding groups, heterocyclic moieties, reporter ligands and peptide motifs;
each of C¹-Cⁿ is (CR⁶R⁷)y where R⁶ is hydrogen and R⁷ is selected from the group consisting of the side chains of naturally occurring alpha amino acids, or R⁶ and R⁷ are independently selected from the group consisting of hydrogen, (C₂-C₆)alkyl, aryl, aralkyl, heteroaryl, hydroxy, (C₁-C₆)alkoxy, (C₁-C₆)alkylthio, NR³R⁴ and SR⁵, where R³ and R⁴ are as defined below, and R⁵ is hydrogen, (C₁-C₆)alkyl or R⁶ and R⁷ taken together complete an alicyclic or heterocyclic system;
each of D¹-Dⁿ is (CR⁶R⁷)_{z} where R⁶ and R⁷ are as defined above;
each of y and z is zero or an integer from 1 to 10, the sum y + z being from 2 to 10;
each of G¹-Gⁿ is -NR³CO-, -NR³C^{S}-, -NR³SO- or -NR³SO₂-, in either orientation, where R³ is as defined below;
each of A¹-Aⁿ and B¹-Bⁿ are selected such that:
(a) A is a group of formula (IIa), (IIb), (IIc) or (IId), and B is N or R³N⁺; or
(b) A is a group of formula (IId) and B is CH; 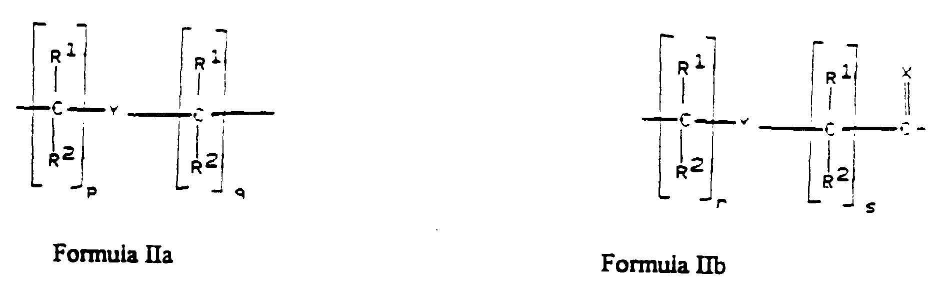 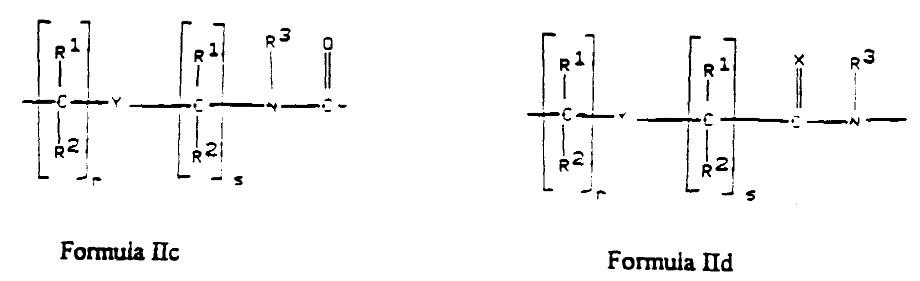
wherein:
X is O, S, Se, NR³, CH₂ or C(CH₃)₂;
Y is a single bond, O, S or NR⁴;
each of p and q is zero or an integer from 1 to 5, the sum p + q being not more than 10;
each of r and s is zero or an integer from 1 to 5, the sum r + s being not more than 10;
each R¹ and R² is independently selected from the group consisting of hydrogen, (C₁-C₄)alkyl which may be hydroxy- or alkoxy- or alkylthio-substituted, hydroxy, alkoxy, alkylthio-substituted, hydroxy, alkoxy, alkylthio, amino and halogen; and
each 3 and R⁴ is independently selected from the group consisting of hydrogen (C₁-C₄)alkyl, hydroxy- or alkoxy- or alkylthio-substituted (C₁-C₄)alkyl, hydroxy, alkoxy, alkylthio and amino;
Q is -CO₂H, -CONR'R", -SO₃H or -SO₂NR'R" or an activated derivative of -CO₂H or -SO₃H; and
1 is -NR'R"', where R' and R" are independently selected from the group consisting of hydrogen, alkyl, amino protecting groups, reporter ligands, intercalators, chelators, peptides, proteins, carbohydrates, lipids, steroids, nucleo-sides, nucleotides, nucleotide diphosphates. nucleotide triphosphates, oligonucleotides, including both oligoribo-nucleotides and
oligodeoxyribonucleotides, oligonucleosides and soluble and non-soluble polymers, and -R"' is an -R" group or the peptide motif, at least one L or the group-R"' being said peptide motif

26. A nucleic acid analogue as claimed in claim 25, wherein said nucleic acid analogue comprises a compound of the general formula III, IV or V: 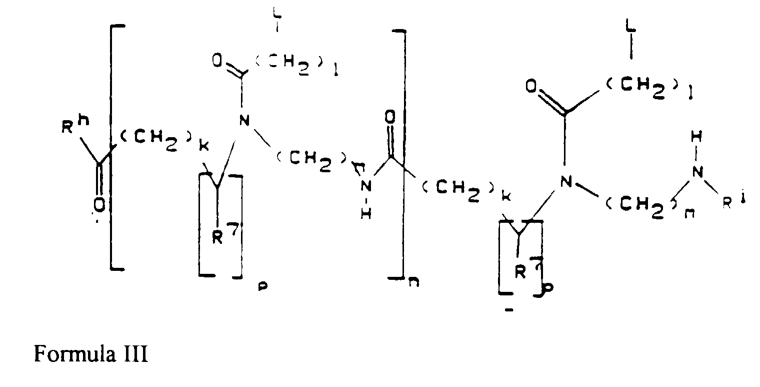 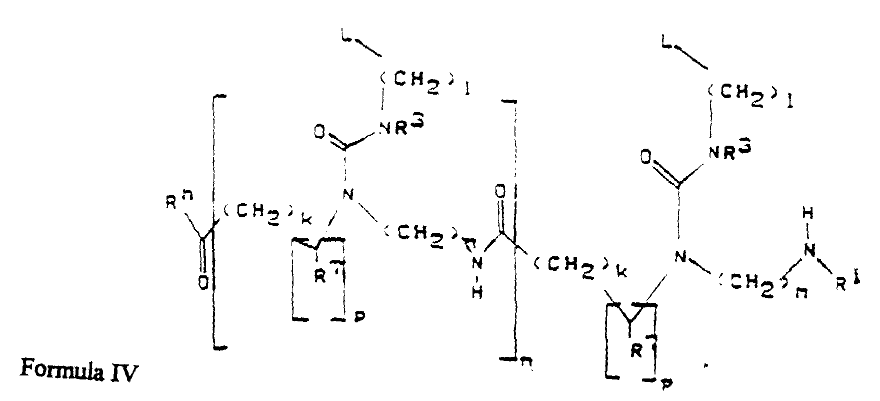 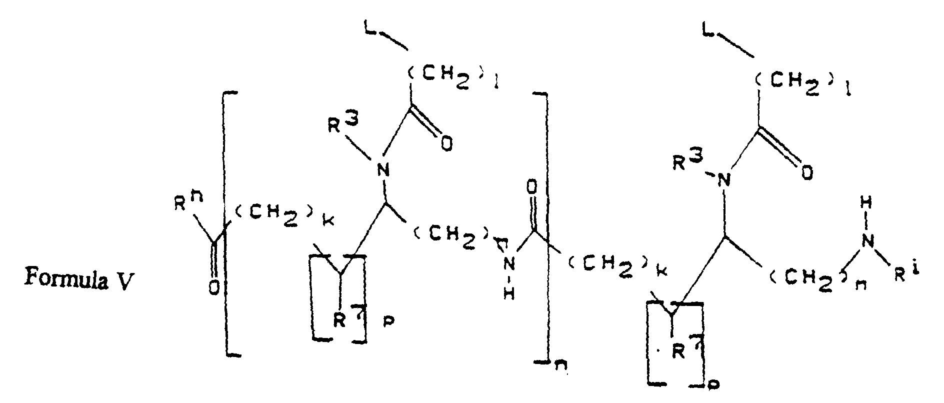 wherein:
each L is independently selected from the group consisting of hydrogen, phenyl, heterocyclic moieties, naturally occurring nucleobases, and non-naturally occurring nucleobases;
each R⁷ is independently selected from the group consisting of hydrogen and the side chains of naturally occurring alpha amino acids;
n is an integer greater than 1,
each k, l, and m is, independently, zero or an integer from 1 to 5;
each p is zero or 1;
R^{h} is OH, NH₂ or -NHLysNH₂; and
Rⁱ is a chelating moiety.

27. An analytical method comprising hybridising a radio-labelled nucleic acid analogue produced by a method as claimed in any one of claims 1 to 15, or as claimed in any one of claims 16 to 26, to a nucleic acid and detecting the presence of the hybrids so produced by the radio-label.

28. A method as claimed in claim 27, wherein the nucleic acid to be detected in bound to a support and is probed using said labelled nucleic acid analogue.

## Patentansprüche

1. Verfahren zum Markieren eines Nucleinsäure-Analogons, umfassend das Bereitstellen eines Nucleinsäure-Analogons mit einer Peptid-Struktur, die als Substrat für ein Enzym in einer Markierungsreaktion fungieren kann, und das Durchführen einer solchen Markierungsreaktion, umfassend das Umsetzen der Peptid-Struktur des Nucleinsäure-Analogons mit einem Ausgangsstoff für die Markierung in einer durch das Enzym vermittelten Reaktion.

2. Verfahren nach Anspruch 1, wobei die Markierung eine Radiomarkierung ist.

3. Verfahren nach Anspruch 1, wobei der Ausgangsstoff für die Markierung radiomarkiertes ATP ist.

4. Verfahren nach Anspruch 3, wobei das Enzym eine Proteinkinase ist.

5. Verfahren nach irgendeinem der vorstehenden Ansprüche, wobei die Peptid-Struktur die Kemptid-Struktur ist.

6. Verfahren nach irgendeinem der Ansprüche 1 bis 5, wobei die Markierungsreaktion eine Phosphorylierung an einem Serin-Rest der Peptid-Struktur ist.

7. Verfahren nach irgendeinem der vorstehenden Ansprüche, wobei das Nucleinsäure-Analogon einen polymeren Strang umfaßt, der eine Sequenz aus Liganden enthält, die an ein Gerüst gebunden sind, das aus verknüpften Gerüsteinheiten besteht, wobei das Analogon imstande ist, an eine Nucleinsäure komplementärer Sequenz zu hybridisieren, und des weiteren diese Peptid-Struktur umfaßt.

8. Verfahren nach Anspruch 7, wobei das Nucleinsäureanalogon-Gerüst ein Polyamid-, Polythioamid-, Polysulfinamid- oder Polysulfonamid-Gerüst ist.

9. Verfahren nach Anspruch 8, wobei die verknüpften Gerüsteinheiten Peptid-gebundene Aminosäure-Einheiten sind.

10. Verfahren nach Anspruch 9, wobei die Peptid-Struktur am N-terminalen Ende oder am C-terminalen Ende vorliegt.

11. Verfahren nach irgendeinem der vorstehenden Ansprüche, wobei das Nucleinsäure-Analogon an eine Nucleinsäure komplementärer Sequenz hybridisieren kann, um ein Hybrid zu bilden, das stabiler gegen Denaturierung durch Wärme ist als ein Hybrid zwischen dem herkömmlichen, in der Sequenz dem Analogon entsprechenden Deoxyribonucleotid und der Nucleinsäure.

12. Verfahren nach irgendeinem der vorstehenden Ansprüche, wobei das Nucleinsäure-Analogon eine Peptidnucleinsäure ist, bei der das Gerüst ein Polyamid-Gerüst ist, wobei jeder Ligand direkt oder indirekt an ein Aza-Stickstoff-Atom im Gerüst gebunden ist, und die Liganden tragenden Stickstoff-Atome vorwiegend durch 4 bis 8 dazwischenliegende Atome voneinander im Gerüst getrennt sind.

13. Verfahren nach irgendeinem der vorstehenden Ansprüche, wobei das Nucleinsäure-Analogon an eine doppelsträngige Nucleinsäure hybridisieren kann, in der ein Strang eine zu dem Analogon komplementäre Sequenz aufweist, um so den anderen Strang von diesem einen Strang zu verdrängen.

14. Verfahren nach irgendeinem der vorstehenden Ansprüche, wobei das Nucleinsäure-Analogon die allgemeine Formel I aufweist: 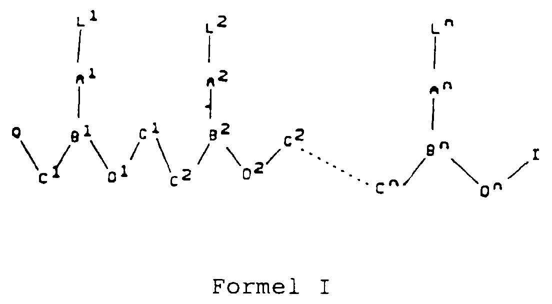 worin:
n wenigstens 2 ist;
jedes der L¹-Lⁿ unabhängig ausgewählt ist aus der Gruppe bestehend aus Wasserstoff, Hydroxy, (C₁-C₄)-Alkanoyl, natürlich vorkommenden Nucleobasen, nicht natürlich vorkommenden Nucleobasen, aromatischen Einheiten, DNA-intercalierenden Agenzien, Nucleobase-bindenden Gruppen, heterocyclischen Einheiten, Reporter-Liganden und der Peptid-Struktur;
jedes der C¹-Cⁿ (CR⁶R⁷)_{y} ist, wobei R⁶ Wasserstoff ist und R⁷ ausgewählt ist aus der Gruppe bestehend aus den Seitenketten natürlich vorkommender α-Aminosäuren, oder R⁶ und R⁷ unabhängig ausgewählt sind aus der Gruppe bestehend aus Wasserstoff, (C₂-C₆)-Alkyl, Aryl, Aralkyl, Heteroaryl, Hydroxy, (C₁-C₆)-Alkoxy, (C₁-C₆)-Alkylthio, NR³R⁴ und SR⁵, wobei R³ und R⁴ wie nachstehend definiert sind, und R⁵ Wasserstoff, (C₁-C₆)-Alkyl, hydroxy-, alkoxy- oder alkylthiosubstituiertes (C₁-C₆)-Alkyl ist, oder R⁶ und R⁷ zusammengenommen ein alicyclisches oder heterocyclisches System vervollständigen;
jedes der D¹-Dⁿ (CR⁶R⁷)_{z} ist, wobei R⁶ und R⁷ wie vorstehend definiert sind;
y und z jeweils null oder eine ganze Zahl von 1 bis 10 sind, wobei die Summe y + z 2 bis 10 ist;
G¹-Gⁿ jeweils -NR³CO-, -NR³CS-, -NR³SO- oder -NR³SO₂- in beiden Orientierungen sind, wobei R³ wie nachstehend definiert ist;
jedes der A¹-Aⁿ und B¹-Bⁿ so ausgewählt ist, daß:
(a) A eine Gruppe der Formel (IIa), (IIb), (IIc) oder (IId) ist, und B N oder R³N⁺ ist; oder
(b) A eine Gruppe der Formel (IId) ist, und B CH ist; 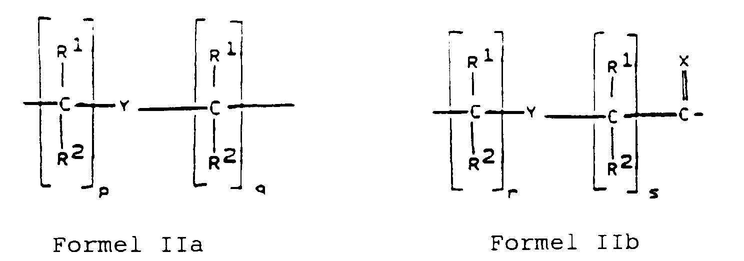 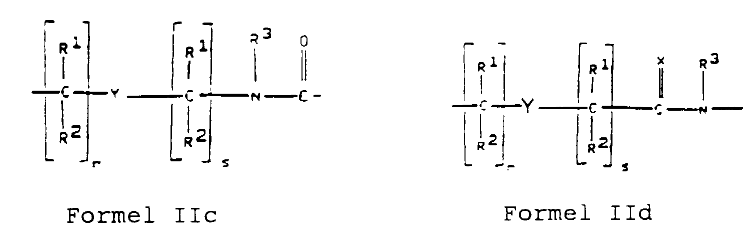
worin:
X O, S, Se, NR³, CH₂ oder C(CH₃)₂ ist;
Y eine Einfachbindung, O, S oder NR⁴ ist;
p und q jeweils null oder ganze Zahlen von 1 bis 5 sind, wobei die Summe p+q nicht größer als 10 ist;
r und s jeweils null oder ganze Zahlen von 1 bis 5 sind, wobei die Summe r+s nicht größer als 10 ist;
R¹ und R² jeweils unabhängig ausgewählt sind aus der Gruppe bestehend aus Wasserstoff, (C₁-C₄)-Alkyl, das hydroxy- oder alkoxy- oder alkylthiosubstituiert sein kann, Hydroxy, Alkoxy, Alkylthio, Amino und Halogen; und
R³ und R⁴ jeweils unabhängig ausgewählt sind aus der Gruppe bestehend aus Wasserstoff, (C₁-C₄)-Alkyl, hydroxy- oder alkoxy- oder alkylthiosubstituiertem (C₁-C₄)-Alkyl, Hydroxy, Alkoxy, Alkylthio und Amino;
Q -CO₂H, -CONR'R", -SO₃H oder -SO₂NR'R" oder ein aktiviertes Derivat von -CO₂H oder -SO₃H ist; und
I -NR'R‴ ist, wobei R' und R" unabhängig ausgewählt sind aus der Gruppe bestehend aus Wasserstoff, Alkyl, Schutzgruppen für Amino, Reporter-Liganden, intercalierenden Agenzien, Chelatbildnern, Peptiden, Proteinen, Kohlenhydraten, Lipiden, Steroiden, Nucleosiden, Nucleotiden, Nucleotiddiphosphaten, Nucleotidtriphosphaten, Oligonucleotiden, darunter sowohl Oligoribonucleotide als auch Oligodeoxyribonucleotide, Oligonucleosiden sowie löslichen und nichtlöslichen Polymeren, und -R‴ eine R"-Gruppe oder die Peptid-Struktur ist, wobei wenigstens L oder -R‴ die Peptid-Struktur ist.

15. Verfahren nach Anspruch 14, wobei das Nucleinsäure-Analogon eine Verbindung der allgemeinen Formel III, IV oder V umfaßt: 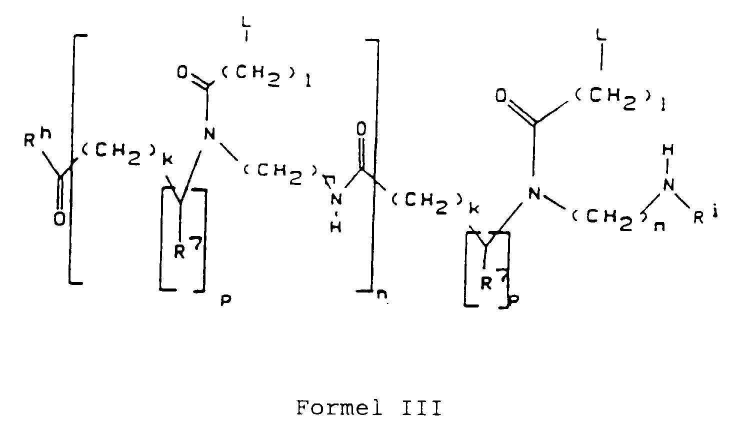 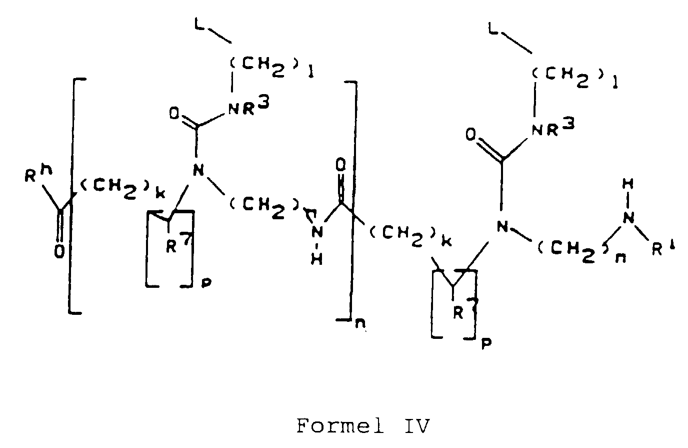 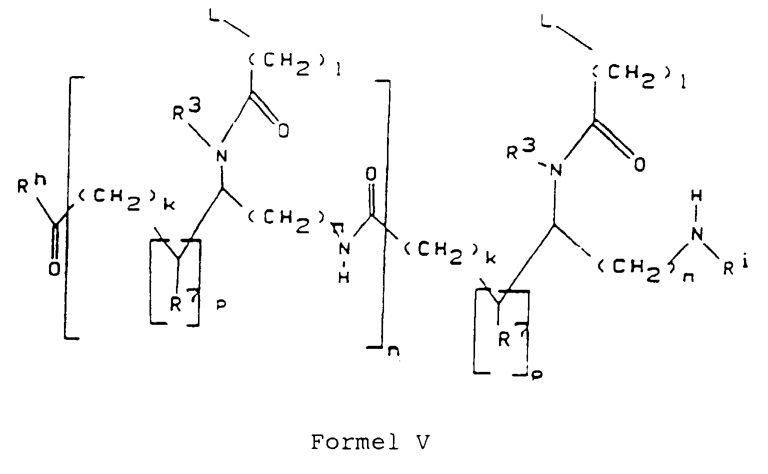 worin:
L jeweils unabhängig ausgewählt ist aus der Gruppe bestehend aus Wasserstoff, Phenyl, heterocyclischen Einheiten, natürlich vorkommenden Nucleobasen und nicht natürlich vorkommenden Nucleobasen;
R⁷ jeweils unabhängig ausgewählt ist aus der Gruppe bestehend aus Wasserstoff und den Seitenketten natürlich vorkommender α-Aminosäuren;
n ein ganze Zahl größer 1 ist;
k, 1 und m jeweils unabhängig null oder ein ganze Zahl von 1 bis 5 sind;
p jeweils null oder 1 ist;
R^{h} OH, NH₂ oder -NHLysNH₂ ist; und
Rⁱ die Peptid-Struktur ist.

16. Nucleinsäure-Analogon, umfassend einen polymeren Strang, der eine Sequenz aus Liganden enthält, die an ein Gerüst gebunden sind, das aus verknüpften Gerüsteinheiten besteht, wobei das Analogon an eine Nucleinsäure komplementärer Sequenz hybridisieren kann, weiterhin umfassend eine Peptid-Struktur, die als Substrat für ein Enzym bei einer Markierungsreaktion fungieren kann.

17. Nucleinsäure-Analogon nach Anspruch 16, wobei die Peptid-Struktur mit radiomarkiertem ATP umgesetzt werden kann, um die Peptid-Struktur in Gegenwart einer Proteinkinase zu phosphorylieren.

18. Nucleinsäure-Analogon nach Anspruch 17, wobei die Peptid-Struktur die Kemptid-Struktur ist.

19. Nucleinsäure-Analogon nach irgendeinem der Ansprüche 16 bis 18, wobei das Gerüst ein Polyamid-, Polythioamid-, Polysulfinamid- oder Polysulfonamid-Gerüst ist.

20. Nucleinsäure-Analogon nach Anspruch 19, wobei die verknüpften Gerüsteinheiten Peptid-gebundene Aminosäure-Einheiten sind.

21. Nucleinsäure-Analogon nach Anspruch 19 oder 20, wobei die Peptid-Struktur am N-terminalen Ende oder am C-terminalen Ende vorliegt.

22. Nucleinsäure-Analogon nach irgendeinem der Ansprüche 16 bis 21, wobei das Nucleinsäure-Analogon an eine Nucleinsäure komplementärer Sequenz hybridisieren kann, um ein Hybrid zu bilden, das stabiler gegen Denaturierung durch Wärme ist als ein Hybrid zwischen dem herkömmlichen, in der Sequenz dem Analogon entsprechenden Deoxyribonucleotid und der Nucleinsäure.

23. Nucleinsäure-Analogon nach irgendeinem der Ansprüche 16 bis 22, wobei das Nucleinsäure-Analogon eine Peptidnucleinsäure ist, bei der das Gerüst ein Polyamid-Gerüst ist, wobei jeder Ligand direkt oder indirekt an ein Aza-Stickstoff-Atom im Gerüst gebunden ist, und die Liganden tragenden Stickstoff-Atome vorwiegend durch 4 bis 8 dazwischenliegende Atome voneinander im Gerüst getrennt sind.

24. Nucleinsäure-Analogon nach irgendeinem der Ansprüche 16 bis 23, wobei das Nucleinsäure-Analogon an eine doppelsträngige Nucleinsäure hybridisieren kann, in der ein Strang eine zu dem Analogon komplementäre Sequenz aufweist, um so den anderen Strang von diesem einen Strang zu verdrängen.

25. Nucleinsäure-Analogon nach irgendeinem der Ansprüche 16 bis 24, wobei das Nucleinsäure-Analogon die allgemeine Formel I aufweist: 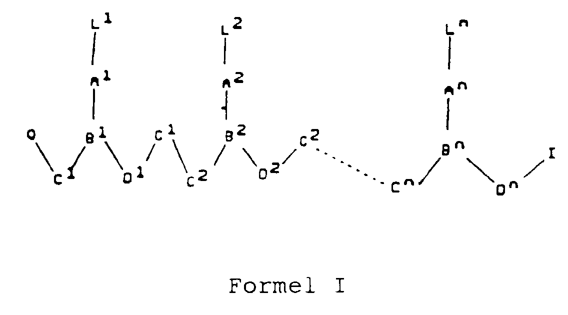 worin:
n wenigstens 2 ist;
jedes der L¹-Lⁿ unabhängig ausgewählt ist aus der Gruppe bestehend aus Wasserstoff, Hydroxy, (C₁-C₄)-Alkanoyl, natürlich vorkommenden Nucleobasen, nicht natürlich vorkommenden Nucleobasen, aromatischen Einheiten, DNA-intercalierenden Agenzien, Nucleobase-bindenden Gruppen, heterocyclischen Einheiten, Reporter-Liganden und Peptid-Strukturen;
jedes der C¹-Cⁿ (CR⁶R⁷)_{y} ist, wobei R⁶ Wasserstoff ist und R⁷ ausgewählt ist aus der Gruppe bestehend aus den Seitenketten natürlich vorkommender α-Aminosäuren, oder R⁶ und R⁷ unabhängig ausgewählt sind aus der Gruppe bestehend aus Wasserstoff, (C₂-C₆)-Alkyl, Aryl, Aralkyl, Heteroaryl, Hydroxy, (C₁-C₆)-Alkoxy, (C₁-C₆)-Alkylthio, NR³R⁴ und SR⁵, wobei R³ und R⁴ wie nachstehend definiert sind, und R⁵ Wasserstoff, (C₁-C₆)-Alkyl ist, oder R⁶ und R⁷ zusammengenommen ein alicyclisches oder heterocyclisches System vervollständigen;
jedes der D¹-Dⁿ (CR⁶R⁷)_{z} ist, wobei R⁶ und R⁷ wie vorstehend definiert sind;
y und z jeweils null oder eine ganze Zahl von 1 bis 10 sind, wobei die Summe y + z 2 bis 10 ist;
G¹-Gⁿ jeweils -NR³CO-, -NR³CS-, -NR³SO- oder -NR³SO₂- in beiden Orientierungen sind, wobei R³ wie nachstehend definiert ist;
jedes der A¹-Aⁿ und B¹-Bⁿ so ausgewählt sind, daß:
(a) A eine Gruppe der Formel (IIa), (IIb), (IIc) oder (IId) ist, und B N oder R³N⁺ ist; oder
(b) A eine Gruppe der Formel (IId) ist, und B CH ist; 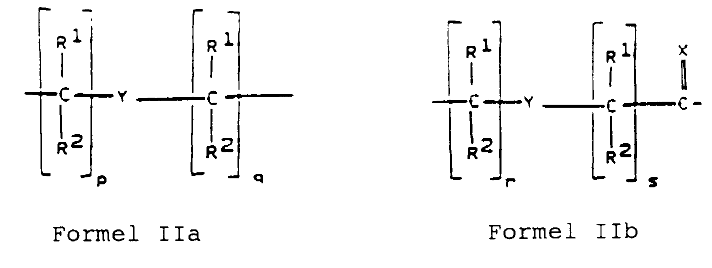 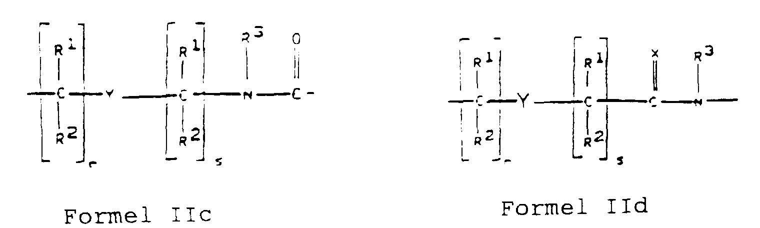
worin:
X O, S, Se, NR³, CH₂ oder C(CH₃)₂ ist;
Y eine Einfachbindung, O, S oder NR⁴ ist;
p und q jeweils null oder ganze Zahlen von 1 bis 5 sind, wobei die Summe p+q nicht größer als 10 ist;
r und s jeweils null oder ganze Zahlen von 1 bis 5 sind, wobei die Summe r+s nicht größer als 10 ist;
R¹ und R² jeweils unabhängig ausgewählt sind aus der Gruppe bestehend aus Wasserstoff, (C₁-C₄)-Alkyl, das hydroxy- oder alkoxy- oder alkylthiosubstituiert sein kann, Hydroxy, Alkoxy, Alkylthio, Amino und Halogen; und
R³ und R⁴ jeweils unabhängig ausgewählt sind aus der Gruppe bestehend aus Wasserstoff, (C₁-C₄)-Alkyl, hydroxy- oder alkoxy- oder alkylthiosubstituiertem (C₁-C₄)-Alkyl, Hydroxy, Alkoxy, Alkylthio und Amino;
Q -CO₂H, -CONR'R", -SO₃H oder -SO₂NR'R" oder ein aktiviertes Derivat von -CO₂H oder -SO₃H ist; und
I -NR'R‴ ist, wobei R' und R" unabhängig ausgewählt sind aus der Gruppe bestehend aus Wasserstoff, Alkyl, Schutzgruppen für Amino, Reporter-Liganden, intercalierenden Agenzien, Chelatbildnern, Peptiden, Proteinen, Kohlenhydraten, Lipiden, Steroiden, Nucleosiden, Nucleotiden, Nucleotiddiphosphaten, Nucleotidtriphosphaten, Oligonucleotiden, darunter sowohl Oligoribonucleotide als auch Oligodeoxyribonucleotide, Oligonucleosiden sowie löslichen und nichtlöslichen Polymeren, und -R‴ eine R"-Gruppe oder die Peptid-Struktur ist, wobei wenigstens ein L oder die Gruppe -R‴ die Peptid-Struktur ist.

26. Nucleinsäure-Analogon nach Anspruch 25, wobei das Nucleinsäure-Analogon eine Verbindung der allgemeinen Formel III, IV oder V umfaßt: 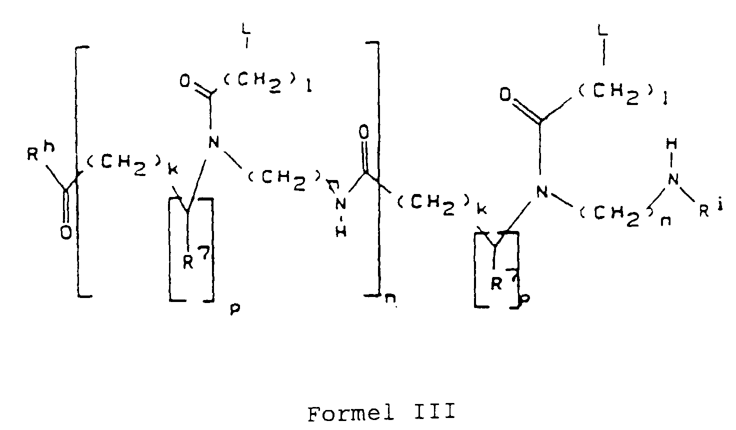 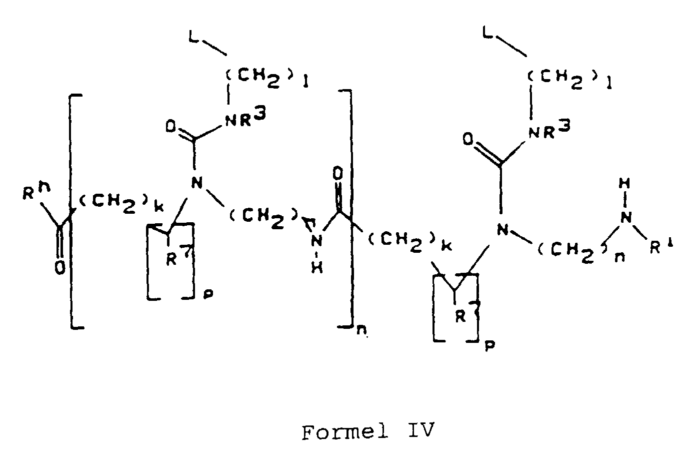 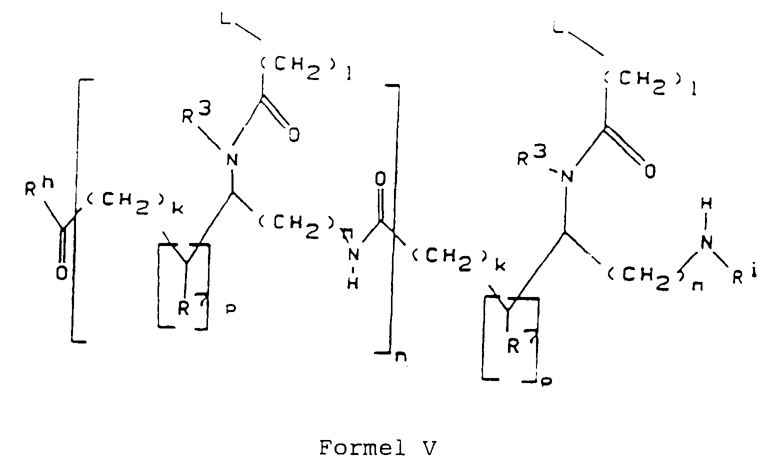 worin:
L jeweils unabhängig ausgewählt ist aus der Gruppe bestehend aus Wasserstoff, Phenyl, heterocyclischen Einheiten, natürlich vorkommenden Nucleobasen und nicht natürlich vorkommenden Nucleobasen;
R⁷ jeweils unabhängig ausgewählt ist aus der Gruppe bestehend aus Wasserstoff und den Seitenketten natürlich vorkommender α-Aminosäuren;
n ein ganze Zahl größer 1 ist;
k, l und m jeweils unabhängig null oder ein ganze Zahl von 1 bis 5 sind;
p jeweils null oder 1 ist;
R^{h} OH, NH₂ oder -NHLysNH₂ ist; und
Rⁱ eine chelatbildende Einheit ist.

27. Analytisches Verfahren, umfassend das Hybridisieren eines radiomarkierten Nucleinsäure-Analogons, hergestellt mit Hilfe eines Verfahrens nach irgendeinem der Ansprüche 1 bis 15 oder nach irgendeinem der Ansprüche 16 bis 26, an eine Nucleinsäure und Nachweisen der Gegenwart des so hergestellten Hybrids mit Hilfe der Radiomarkierung.

28. Verfahren nach Anspruch 27, wobei die nachzuweisende Nucleinsäure an einen Träger gebunden ist und mit dem markierten Nucleinsäure-Analogon geprüft wird.

## Revendications

1. Procédé pour marquer un analogue d'acide nucléique consistant à fournir un analogue d'acide nucléique avec un motif peptide capable de réagir en tant que substrat dans une réaction de marquage et à réaliser une réaction dite de marquage consistant à faire réagir le motif peptide de l'analogue d'acide nucléique dans une réaction régulée par ladite enzyme avec une source de marquage.

2. Procédé selon la revendication 1, dans lequel ladite marque est une radiomarque

3. Procédé selon la revendication 1, dans lequel la source de ladite marque est un ATP radiomarqué.

4. Procédé selon la revendication 3, dans lequel ladite enzyme est une protéine kinase.

5. Procédé selon l'une quelconque des revendications précédentes, dans lequel le motif peptide est le motif kemptide.

6. Procédé selon l'une quelconque des revendications 1 à 5, dans lequel la réaction de marquage est une phosphorylation au niveau d'un résidu sérine dudit motif peptide.

7. Procédé selon l'une quelconque des revendications précédentes, dans lequel l'analogue d'acide nucléique comprend un brin polymère qui inclut une séquence de ligands liés à un squelette fait de fractions de squelette liées, analogue qui est capable de s'hybrider à un acide nucléique de séquence complémentaire, et qui comprend de plus ledit motif peptide.

8. Procédé selon la revendication 7, dans lequel ledit squelette d'analogue d'acide nucléique est un squelette polyamide, polythioamide, polysulfinamide ou polysulfonamide.

9. Procédé selon la revendication 8, dans lequel lesdites fractions de squelette liées sont des fractions d'aminoacide liées à un peptide.

10. Procédé selon la revendication 9, dans lequel ledit motif peptide est présent au niveau de la terminaison C.

11. Procédé selon l'une quelconque des revendications précédentes, dans lequel l'analogue d'acide nucléique est capable de s'hybrider à un acide nucléique de séquence complémentaire pour former un hybride qui est plus stable envers une dénaturation par la chaleur qu'un hybride entre le déoxyribonucléotide classique correspondant en séquence audit analogue et audit acide nucléique.

12. Procédé selon l'une quelconque des revendications précédentes, dans lequel ledit analogue d'acide nucléique est un acide nucléique peptidique dans lequel ledit squelette est un squelette polyamide, chaque dit ligand étant lié directement ou indirectement à un atome d'azote aza dans ledit squelette, et lesdits atomes d'azote portant un ligand étant principalement séparés les uns des autres dans ledit squelette par 4 à 8 atomes intervenants.

13. Procédé selon l'une quelconque des revendications précédentes, dans lequel l'analogue d'acide nucléique est capable de s'hybrider à un acide nucléique double-brin dans lequel un brin possède une séquence complémentaire audit analogue, de façon à déplacer l'autre brin dudit un brin.

14. Procédé selon l'une quelconque des revendications précédentes, dans lequel l'analogue d'acide nucléique possède la formule générale 1: 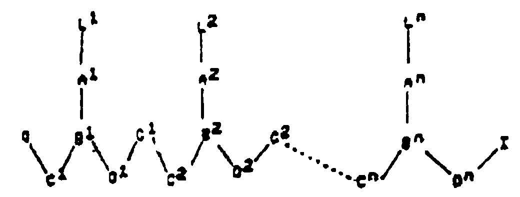 dans laquelle:
n vaut au moins 2,
chacun de L¹ à Lⁿ est choisi indépendamment dans le groupe formé par un atome d'hydrogène, un groupe hydroxy, alcanoyle en C₁ à C₄, les nucléobases d'origine naturelle, les nucléobases d'origine non naturelle, les fractions aromatiques, les intercaleurs d'ADN, les groupes liant des nucléobases, les fractions hétérocycliques, les ligands reporteurs et ledit motif peptide;
chacun de C¹ à Cⁿ représente (CR⁶CR⁷)_{y} où R⁶ est un atome d'hydrogène et R⁷ est choisi dans le groupe formé par les chaînes secondaires des alpha-aminoacides d'origine naturelle, ou R⁶ et R⁷ sont choisis indépendamment dans le groupe formé par un atome d'hydrogène, les groupes alkyle en C₂ à C₆, aryle, aralkyle, hétéroaryle, hydroxy, alcoxy en C₁ à C₆, alkylthio en C₁ à C₆, NR³R⁴ et ^{SR}, où R³ et R⁴ sont tels que définis ci-dessous, et R⁵ représente un atome d'hydrogène, un groupe alkyle en C₁ à C₆, hydroxy, alcoxy, ou alkyle en C₁ à C₆ substitué par un alkylthio, ou R⁶ et R⁷ pris ensemble complètent un système alicyclique ou hétérocyclique;
chacun de D¹ à Dⁿ représente (CR⁶R⁷)_{z} où R⁶ et R⁷ sont tels que définis ci-dessus;
chacun de y et z vaut zéro ou est un nombre entier de 1 à 10, la somme y+z valant de 2 à 10;
chacun de G¹ à Gⁿ représente -NR³CO-, -NR³CS-, -NR³SO- ou -NR³SO₂-, dans toutes les orientations, où R³ est tel que défini ci-dessous;
chacun de A¹ à Aⁿ et de B¹ à Bⁿ sont choisis de sorte que:
(a) A soit un groupe de formule (IIa), (IIb), (IIc) ou (IId), et B représente N ou R³N⁺; ou
(b) A soit un groupe de formule (IId) et B représente CH; 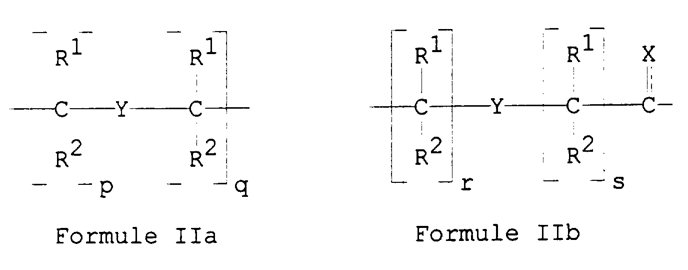 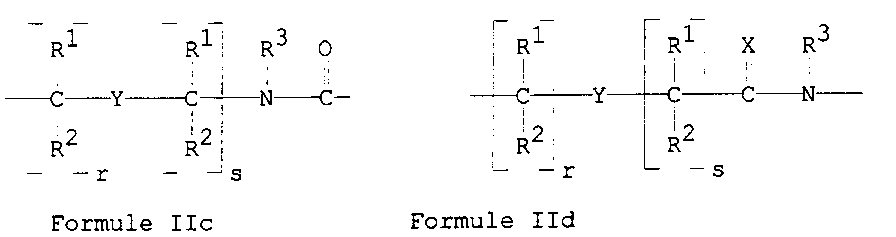
dans lesquelles
X représente O, S, Se, NR³, CH₂ ou C(CH₃)₂;
Y est une liaison simple, O, S, ou NR⁴;
chacun de p et q vaut zéro ou est un nombre entier de 1 à 5, la somme p+q ne dépassant pas 10;
chacun de r et s vaut zéro ou est un nombre entier de 1 à 5, la somme r+s ne dépassant pas 10;
chacun de R¹ et R² est choisi indépendamment dans le groupe formé par un atome d'hydrogène, un groupe alkyle en C₁ à C₄ (qui peut être substitué par un groupe hydroxy, alcoxy ou alkylthio), hydroxy, alcoxy, alkylthio, amino et halogène; et
chacun de R³ et R⁴ est choisi indépendamment dans le groupe formé par un atome d'hydrogène, un groupe alkyle en C₁ à C₄, alkyle en C₁ à C₄ substitué par un groupe hydroxy, alcoxy ou alkylthio, hydroxy, alcoxy, alkylthio et amino;
Q représente -CO₂H,-CONR'R", -SO₃H ou -SO₂NR'R" ou un dérivé activé de -CO₂H ou -SO₃H; et
I représente -NR'R"', où R' et R" sont choisis indépendamment dans le groupe formé par un atome d'hydrogène, un groupe alkyle, les groupes amino-protecteurs, les ligands reporteurs, les intercaleurs, les agents de chélation, les peptides, les protéines, les sucres, les lipides, les stéroïdes, les nucléosides, les nucléotides, les nucléotide-diphosphates, les nucléotide-triphosphates, les oligonucléotides, comprenant à la fois les oligoribonucléotides et les oligodéoxyribonucléotides, les oligonucléosides et les polymères solubles et insolubles,
et -R‴ est un groupe R" ou dudit motif peptide. au moins L ou -R"' représentant ledit motif peptide.

15. Procédé selon la revendication 14, dans lequel ledit analogue d'acide nucléique comprend un composé de formule générale III, IV ou V: 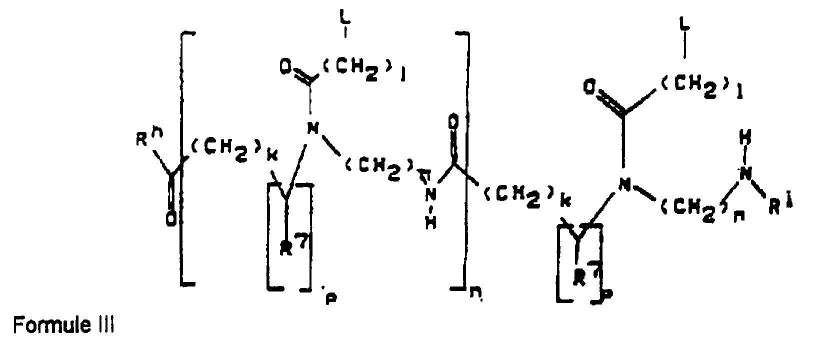 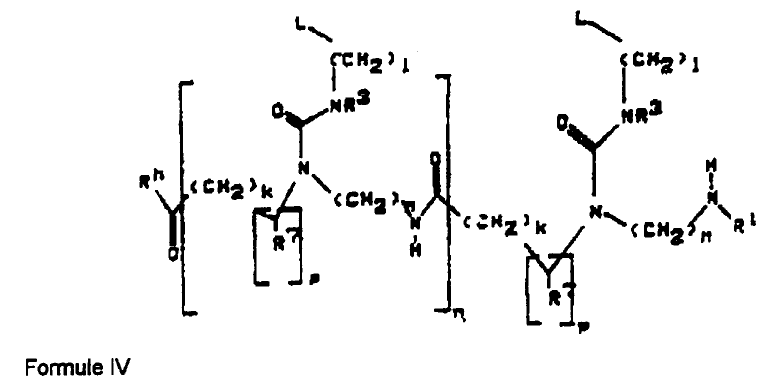 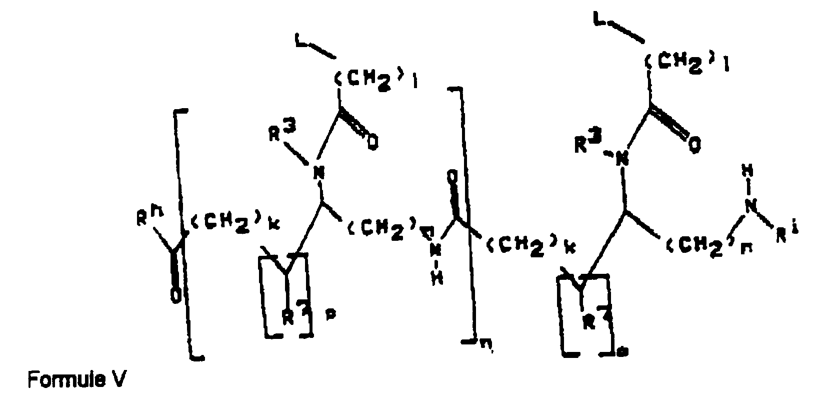 dans lesquelles:
chaque L est choisi indépendamment dans le groupe formé par un atome d'hydrogène, un groupe phényle, les fractions hétérocycliques, les nucléobases d'origine naturelle, et les nucléobases d'origine non naturelle;
chaque R⁷ est choisi indépendamment dans le groupe formé par un atome d'hydrogène et les chaînes latérales des alpha-aminoacides d'origine naturelle;
n est un nombre entier supérieur à 1,
chacun de k, l, et m, vaut, indépendamment, zéro ou est un nombre entier de 1 à 5;
chaque p vaut 0 ou 1;
R^{h} représente OH, NH₂ ou -NHLysNH₂; et
Rⁱ est un motif peptide.

16. Analogue d'acide nucléique comprenant un brin polymère qui comprend une séquence de ligands liés à un squelette fait de fractions de squelette liées, analogue qui est capable de s'hybrider à un acide nucléique de séquence complémentaire, comprenant de plus un motif peptide capable d'agir en tant que substrat pour une enzyme dans une réaction de marquage.

17. Analogue d'acide nucléique selon la revendication 16, dans lequel ledit motif peptide peut réagir avec un ATP radiomarqué pour phosphoryler ledit motif peptide en présence d'une protéine kinase.

18. Analogue d'acide nucléique selon la revendication 17, dans lequel le motif peptide est le motif kemptide.

19. Analogue d'acide nucléique selon l'une quelconque des revendications 16 à 18, dans lequel le squelette est un squelette polyamide, polythioamide, polysulfinamide, ou polysulfonamide.

20. Analogue d'acide nucléique selon la revendication 19, dans lequel lesdites fractions de squelette liées sont des fractions d'aminoacide liées à un peptide.

21. Analogue d'acide nucléique selon la revendication 19 ou la revendication 20, dans lequel ledit motif peptide est présent au niveau de l'extrémité N ou est présent au niveau de la terminaison C.

22. Analogue d'acide nucléique selon l'une quelconque des revendications 16 à 21, dans lequel ledit analogue d'acide nucléique est capable de s'hybrider à un acide nucléique de séquence complémentaire pour former un hybride qui est plus stable envers une dénaturation par la chaleur qu'un hybride entre le déoxyribonucléotide classique correspondant en séquence audit analogue et audit acide nucléique.

23. Analogue d'acide nucléique selon l'une quelconque des revendications 16 à 22, dans lequel l'analogue d'acide nucléique est un acide nucléique peptidique dans lequel ledit squelette est un squelette polyamide, chaque dit ligand étant directement ou indirectement relié à un atome d'azote aza dans ledit squelette, et lesdits atomes d'azote portant un ligand étant principalement séparés les uns des autres dans ledit squelette par 4 à 8 atomes intervenants.

24. Analogue d'acide nucléique selon l'une quelconque des revendications 16 à 23, dans lequel l'analogue d'acide nucléique est capable de s'hybrider à un acide nucléique double-brin dans lequel un premier brin possède une séquence complémentaire audit analogue, de façon à déplacer l'autre brin dudit premier brin.

25. Analogue d'acide nucléique selon l'une quelconque des revendications 16 à 24, dans lequel l'analogue d'acide nucléique possède la formule générale I: 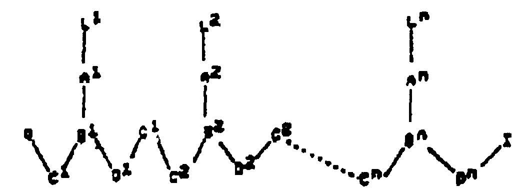 dans laquelle:
n vaut au moins 2
chacun de L¹ à Lⁿ est choisi indépendamment dans le groupe formé par un atome d'hydrogène, un groupe hydroxy, alcanoyle en C₁ à C₄, les nucléobases d'origine naturelle, les nucléobases d'origine non naturelle, les fractions aromatiques, les intercaleurs d'ADN, les groupes liant les nucléobases, les fractions hétérocycliques, les ligands reporteurs et les motifs peptides;
chacun de C¹ à Cⁿ représente (CR⁶R⁷)_{Y} où R⁶ représente un atome d'hydrogène et R⁷ est choisi dans le groupe formé par les chaînes latérales d'alpha-aminoacides d'origine naturelle, ou R⁶ et R⁷ sont choisis indépendamment dans le groupe formé par un atome d'hydrogène, un groupe alkyle en C₂ à C₆, aryle, aralkyle, hétéroaryle, hydroxy, alcoxy en C₁ à C₆, alkylthio en C₁ à C₆, NR³R⁴ et ^{SR}, où R³ et R⁴ sont tels que définis ci-dessous, et R⁵ est un atome d'hydrogène, un groupe alkyle en C₁ à C₆ ou R⁶ et R⁷ pris ensemble complètent un système alicyclique ou hétérocyclique;
chacun de D¹ à Dⁿ représente (CR⁶R⁷)_{y} où R⁶ et R⁷ sont tels que défini ci-dessus;
chacun de y et de z vaut zéro ou est un nombre entier de 1 à 10, la somme y+z valant de 2 à 10;
chacun de ^{Ge} à ^{GMt} représente -NR³CO-, -NR³CS-, -NR³SO- ou -NR³SO₂, dans toutes les orientations, où R³ est tel que défini ci-dessous,
chacun de A¹ à Aⁿ et de B¹ à Bⁿ sont choisis de sorte que:
(a) A soit un groupe de formule (IIa), (IIb), (IIc) ou (IId), et B représente N ou R³N⁺; ou
(b) A soit un groupe de formule (IId) et B représente CH; 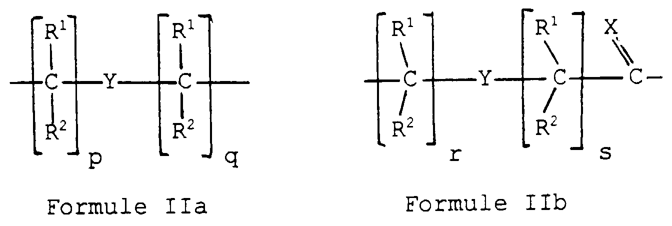 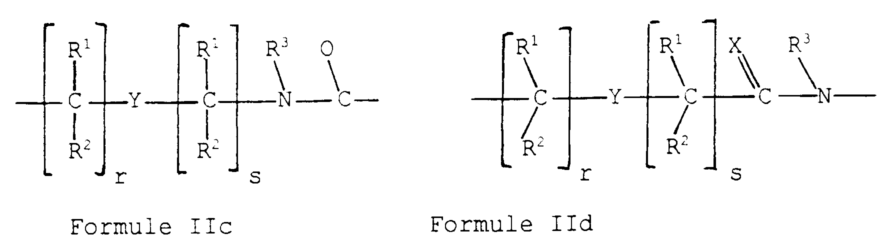 dans lesquelles:
X représente O, S, Se, NR³, CH₂ ou C(CH₃)₂
Y est une liaison simple, O, S ou NR⁴;
chacun de p et q vaut zéro ou est un nombre entier de 1 à 5, la somme p+q ne valant pas plus de 10;
chacun de r et s vaut zéro ou est un nombre entier compris entre 1 et 5, la somme r+s ne dépassant pas 10;
chacun de R¹ et R² est choisi indépendamment dans le groupe formé par un atome d'hydrogène, un groupe alkyle en C₁ à C₄ (qui peut être substitué par un groupe hydroxy ou alcoxy ou alkylthio), hydroxy, alkylthio amino et halogène; et
chacun de R³ et R⁴ est choisi indépendamment dans le groupe formé par un atome d'hydrogène, un groupe alkyle en C₁ à C₄, alkyle en C₁ à C₄ substitué par un groupe hydroxy, alcoxy ou alkylthio, hydroxy, alcoxy, alkylthio et amino.
Q représente -CO₂H, -CONR'R", -SO₃H ou -SO₂NR'R" ou un dérivé activé de -CO₂H ou -SO₃H; et
I représente -NR'R"', où R' et R" sont choisis indépendamment dans le groupe formé par un atome d'hydrogène, un groupe alkyle, les groupes amino-protecteurs, les ligands reporteurs, les intercaleurs, les agents de chélation, les peptides, les protéines, les sucres, les lipides, les stéroïdes, les nucléosides, les nucléotides, les nucléotide-diphosphates, les nucléotide-triphosphates, les oligonucléotides, comprenant à la fois les oligoribonucléotides et les oligodéoxyribonucléotides, les oligonucléosides et les polymères solubles et non solubles, et -R"' est un groupe R" ou le motif peptide, au moins un L ou le groupe R"' étant ledit motif peptide.

26. Analogue d'acide nucléique selon la revendication 25, dans lequel ledit analogue d'acide nucléique comprend un composé de formule générale III, IV, ou V: 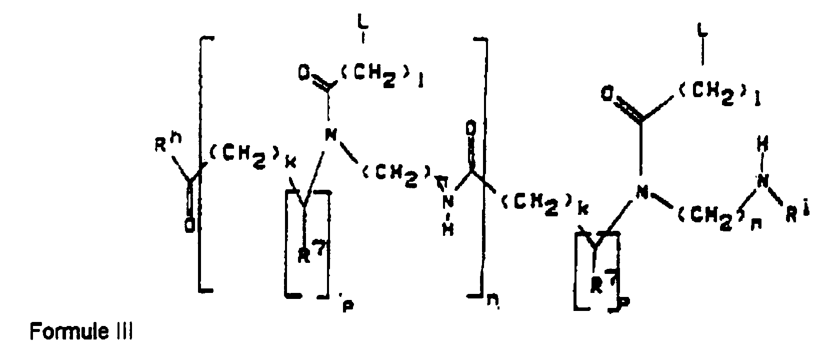 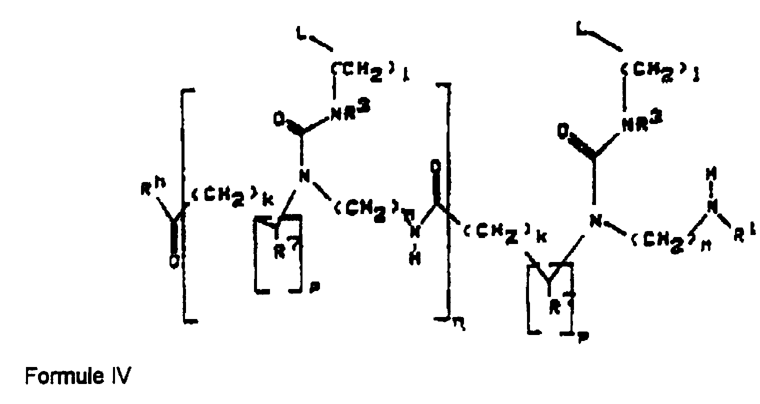 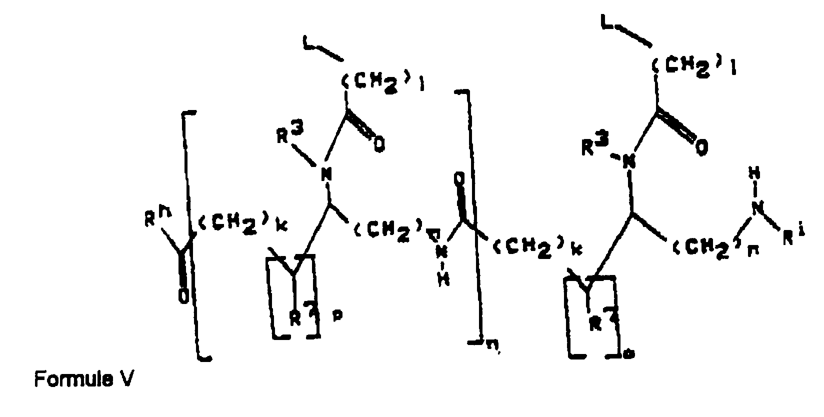 dans lesquelles:
chaque L est choisi indépendamment dans le groupe formé par un atome d'hydrogène, un groupe phényle, les fractions hétérocycliques, les nucléobases d'origine naturelle et les nucléobases d'origine non naturelle;
chaque R⁷ est choisi indépendamment dans le groupe formé par un atome d'hydrogène et les chaînes latérales d'alpha-aminoacides d'origine naturelle;
n est un nombre entier supérieur à 1,
chaque k, l et m vaut indépendamment, zéro ou est un nombre entier de 1 à 5;
chaque p vaut 0 ou 1;
R^{h} représente OH, NH₂, ou -NHLysNH₂; et
Rⁱ est une fraction de chélation.

27. Procédé analytique comprenant l'hybridation d'un analogue d'acide nucléique radiomarqué produit à l'aide d'un procédé selon l'une quelconque des revendications 1 à 15, ou selon l'une quelconque des revendications 16 à 26, à un acide nucléique et la détection de la présence des hybrides ainsi produits par le radiomarquage.

28. Procédé selon la revendication 27, dans lequel l'acide nucléique à détecter est lié à un support et est détecté en utilisant ledit analogue d'acide nucléique marqué.
